# EUROPEAN PATENT APPLICATION

(11) **EP 4 242 317 A1**
(43) Date of publication of application: **13.09.2023**
(21) Application number: 21889268.5
(22) Date of filing: 05.11.2021
(51) Int. Cl.: C12N 15/86, C12N 5/10

(54) **METHOD FOR PRODUCING REVERSIBLY IMMORTALIZED CELL**

(30) Priority: 06.11.2020 JP 2020186146
(71) Applicant: Trans Chromosomics, Inc., Yonago-shi, Tottori 683-8503 (JP); National University Corporation Tottori University, Tottori-shi, Tottori 680-8550 (JP)
(72) Inventor: OSHIMURA, Mitsuo, Yonago-shi, Tottori 683-8503 (JP); TABATA, Toshiaki, Tsukuba-shi, Ibaraki 300-2611 (JP); KAZUKI, Yasuhiro, Yonago-shi, Tottori 683-8503 (JP); UNO, Narumi, Hino-shi, Tokyo 191-0043 (JP)
(74) Representative: D Young & Co LLP
(86) International application number: PCT/JP2021/040757
(87) International publication number: WO 2022/097716

(57) **Abstract**

An object of the present invention is to provide a method for producing a reversibly immortalized cell which can allow a cell into which an immortalizing gene is introduced to proliferate over a long period without damaging the chromosome of the cell, and is capable of removing the immortalizing gene, and to provide a method for obtaining a large amount of a reversibly immortalized cell that can be cloned and has stable quality. The present invention provides a method for producing a reversibly immortalized cell, comprising the steps of introducing a chromosomally non-integrated RNA virus vector loaded with one or two or more immortalizing gene(s), selected from the group consisting of Bmi-1 gene, TERT gene, and SV40T gene, into a mammalian cell so that the immortalizing gene is expressed in the cell; and culturing the obtained cell for proliferation.

## Description

### Technical Field

The present invention relates to a method for producing a reversibly immortalized cell and more specifically relates to a method for producing a reversibly immortalized cell using a chromosomally non-integrated RNA virus vector loaded with a predetermined immortalizing gene.

### Background Art

Somatic stem cells such as mesenchymal stem cells (MSCs), and pluripotent stem cells such as ES cells or iPS cells are used as main cell sources for use in regenerative medicine or cell therapy. Among the stem cells, the mesenchymal stem cells (MSCs) are cells originally present in the body and are therefore excellent in safety because of less risk of rejection or tumorigenic transformation. However, a challenge to these MSCs is to secure a sufficient number of cells necessary for supply to medical setting due to a limited number of cell division rounds.

Hence, an attempt has been made to introduce immortalizing genes into cells for infinite proliferation. Telomerase reverse transcriptase (TERT) gene, genes that regulate the expression or activity of telomerase (e.g., Myc gene and Ras gene), and virus genes (SV40T, HPV E6-E7, EBV, etc.) are known as the immortalizing genes. The introduction of these immortalizing genes into cells employ a vector such as plasmid DNA, a lentivirus vector, a retrovirus vector, or an adenovirus vector (Patent Literatures 1, 2, and 3, etc.). The virus vector is preferred because efficient gene introduction can be achieved by merely filtering a culture supernatant of recombinant virus vector-producing cells and adding the resultant to the cells of interest. Among the vectors described above, the plasmid DNA or the adenovirus vector has DNA as the vector genome and is integrated in the chromosome of a host. The lentivirus vector or the retrovirus vector has RNA as the vector genome and however, assumes the form of DNA by reverse transcriptase in cells and is integrated in the chromosome of a host so that a gene loaded therein is expressed. Such a vector generally used in immortalization has the risk of damage on genes of host cells due to the chromosomal integration of foreign DNA in the host cells, and in some cases, tumorigenic transformation, and therefore presents a significant problem associated with the safety of regenerative medicine. On the other hand, a Sendai virus vector (SeV vector) is a chromosomally non-integrated RNA virus vector which does not assume the form of DNA. The SeV vector permits gene introduction into a wide range of cell types and facilitates high expression of a protein, and as such, is routinely used in the induction of iPS cells, etc. (Patent Literature 4). However, in the case of cells, such as mesenchymal stem cells (MSCs), which have a limited number of division rounds and arrests their division as two months or longer pass, the SeV vector are recognized as having the difficulty in the expression of a transgene for a long period. Therefore, use of the SeV vector is limited to transient high expression, and this vector has not been used in the introduction of immortalizing genes into cells.

Meanwhile, research has been made on a reversibly (conditionally) immortalized cell which is prepared by immortalizing a cell through introduction of an immortalizing gene which may in turn be cleaved using site-specific recombinase after proliferation (Non Patent Literature 1, etc.). However, even if the immortalizing gene is cleaved using site-specific recombinase, the obtained cell is a genetically manipulated cell, and such a cell might have adverse influence such as tumor formation on a host and does not secure safety.

Stem cells obtained from a human tissue are a mixed population of young cells and senescent cells. Since these stem cells vary in properties and together become senescent with increase in the number of division rounds, the cells cannot be maintained with constant quality. Hence, another challenge thereto is difficult setting of the standard of quality which is a factor necessary for automation or mechanization.

Discussion has also been started about an attempt to separate only cells having the properties of interest from such a population of different cells. miRNA analysis, surface marker detection, image analysis of cell morphology, and epigenomic analysis are known as methods for evaluating the quality of iPS cells, and use of these techniques has also been proposed for MSCs. Unlike iPS cells, MSCs neither proliferate infinitely nor can be cloned. Therefore, such use has not been practically carried out.

In the case of separating MSCs having a small passage number without increase in the number of division rounds, a large amount of a tissue containing MSCs is necessary, and a necessary number of cells is also difficult to secure. These factors are responsible for steep rise in production cost. Hence, therapy that requires a large number of cells is difficult, and autologous cell therapy is mainly practiced. In these circumstances, inexpensive regenerative medicine with allogeneic cells is difficult to carry out.

### Citation List

### Patent Literature

Patent Literature 1: JP Patent No. 3953399
Patent Literature 2: WO2017/078176
Patent Literature 3: JP Patent Publication (Kokai) No. 2017-221219 A (2017)
Patent Literature 4: JP Patent No. 5763340

### Non Patent Literature

Non Patent Literature 1: Fang-Ying Meng, et al., Reversible immortalization of human hepatocytes mediated by retroviral transfer and site-specific recombination. World Journal of Gastroenterology, 01 Sep 2014, 20 (36): 13119-13126

### Summary of Invention

### Technical Problem

An object of the present invention is to provide a method for producing a reversibly immortalized cell which can allow a cell into which an immortalizing gene is introduced to proliferate over a long period without damaging the chromosome of the cell, and is capable of removing the immortalizing gene, and to provide a method for obtaining a large amount of a reversibly immortalized cell that can be cloned and has stable quality.

### Solution to Problem

The present inventors have conducted diligent studies to attain the object and consequently found that, when a Sendai virus vector, a chromosomally non-integrated RNA vector which had not previously been used in the immortalization of somatic cells, was loaded with one or two or more immortalizing gene(s) selected from the group consisting of Bmi-1 gene, TERT gene, and SV40T gene, and used in the introduction into mesenchymal stem cells (MSCs), surprisingly, the infinite proliferation (immortalization) of the cells was achieved without causing cell division arrest for 80 days or longer; and a cell proliferation rate was elevated at an early stage after gene introduction. The prepared immortalized cells have also been confirmed to have no chromosomal abnormality, to have multilineage potential, and to enable the immortalizing gene to be easily removed by temperature control. The present inventors have further successfully cloned immortalized cells from the obtained immortalized cell population. As for the cloned immortalized cells, it has also been confirmed that: 8 out of 10 clones exhibited a normal karyotype and had multilineage potential; and the immortalization of cells of any stage from young cells to senescent cells which arrested their division was able to be induced, and the cells were able to be cloned immediately after immortalization. The present invention has been completed on the basis of these findings.

Specifically, the present invention encompasses the following aspects.
[1] A method for producing a reversibly immortalized cell, comprising the steps of
   (1) introducing a chromosomally non-integrated RNA virus vector loaded with an immortalizing gene into a mammalian cell so that the immortalizing gene is expressed in the cell; and
   (2) culturing the cell obtained in the step (1) for proliferation.
[2] The method according to [1], wherein the immortalizing gene is one or two or more immortalizing gene(s) selected from the group consisting of Bmi-1 gene, TERT gene, and SV40T gene.
[3] The method according to [1] or [2], wherein the immortalizing gene is any of the following (a) to (d):
   (a) a combination of Bmi-1 gene, TERT gene, and SV40T gene;
   (b) a combination of Bmi-1 gene and TERT gene;
   (c) a combination of TERT gene and SV40T gene; and
   (d) TERT gene.
[4] The method according to any of [1] to [3], wherein the cell is a somatic cell.
[5] The method according to [4], wherein the somatic cell is a somatic stem cell.
[6] The method according to [5], wherein the somatic stem cell is a mesenchymal stem cell.
[7] The method according to any of [1] to [6], wherein the chromosomally non-integrated RNA virus vector is a negative-strand RNA virus vector.
[8] The method according to [7], wherein the negative-strand RNA virus vector is a paramyxovirus vector.
[9] The method according to [8], wherein the paramyxovirus vector is a Sendai virus vector.
[10] The method according to [9], wherein the Sendai virus vector is a temperature-sensitive Sendai virus vector.
[11] The method according to [1], wherein the chromosomally non-integrated RNA virus vector is a Sendai virus vector, and the method further comprises the step of removing the Sendai virus vector after culture in the step (2).
[12] The method according to [11], wherein the removal of the Sendai virus vector is performed by changing a culture temperature from 35°C to 37°C.
[13] The method according to any of [1] to [12], further comprising the step of cloning the immortalized cell after culture in the step (2).
[14] An immortalized cell obtainable by a method according to any of [1] to [13].
[15] An immortalized cell comprising a removable state of a Sendai virus vector loaded with one or two or more immortalizing gene(s) selected from the group consisting of Bmi-1 gene, TERT gene, and SV40T gene.
[16] A regenerative medicine product comprising an immortalized cell according to [14] or [15].
[17] A temperature-sensitive Sendai virus vector loaded with one or two or more immortalizing gene(s) selected from the group consisting of Bmi-1 gene, TERT gene, and SV40T gene.
[18] A kit for reversibly immortalized cell preparation comprising a temperature-sensitive Sendai virus vector according to [17].

The present application claims the priority of Japanese Patent Application No. 2020-186146 filed on November 6, 2020, and encompasses the contents described in the specification of the patent application.

### Advantageous Effects of Invention

The present invention enables cells difficult to culture in a large amount, for example, mesenchymal stem cells (MSCs) for use in regenerative medicine, to be immortalized (to proliferate infinitely) without damaging the chromosome thereof, by introducing an immortalizing gene into the cells using a chromosomally non-integrated RNA vector. This immortalization is achieved, irrespective of the number of division rounds of the cells to be immortalized and the number of cell division rounds upon immortalization. Use of a temperature-sensitive Sendai virus vector (SeV vector) as the chromosomally non-integrated RNA vector enables the vector to be easily removed from the cells by mere change of a culture temperature without a complicated removal operation. Accordingly, cells excellent in safety can be supplied in a large amount. The immortalized cell obtained by the present invention has no chromosomal abnormality and has multilineage potential, as in before gene introduction, and as such, can be used in regenerative medicine. Unimmortalized cells are a population of cells differing in lifetime or properties and are therefore difficult to clone. On the other hand, the immortalized cell prepared by the present invention is easy to clone, and cells having high quality can be stably obtained. Therefore, the immortalized cell prepared by the present invention facilitates quality management by the mechanization of cell production, is capable of drastically reducing production cost which is a challenge to industrialization, and can contribute to the advancement of cell medicine or regenerative medicine.

### Brief Description of Drawings

[Figure 1] Figure 1 shows the genomic structure of a SeV vector loaded with an immortalizing factor gene [A. SeV(18+)Bmi1(HNL)OFP/TS15dΔF, B. SeV(PM)hTERT(HNL)EGFP/TS15ΔF, C. SeV(18+)SV40T/TS15ΔF, D. SeV(HNL)E6-E7, BFP/TS15ΔF].
[Figure 2] Figure 2 shows transmitted light images and fluorescent (OFP) images of cells infected with Bmi-1 alone (MOI: 1, 5, and 20), and transmitted light images and fluorescent (GFP) images of cells infected with hTERT alone (MOI: 1, 5, and 20).
[Figure 3] Figure 3 shows transmitted light images and fluorescent (OFP and GFP) images of cells coinfected with two factors (Bmi-1/hTERT) (MOI: 1, 5, and 20).
[Figure 4] Figure 4 shows transmitted light images and fluorescent (OFP and GFP) images of cells coinfected with three factors (Bmi-1/hTERT/SV40T) (MOI: 1, 5, and 20).
[Figure 5] Figure 5 shows the rating of the morphology and state of cells 59 days after introduction of immortalizing factors (No. 1 to No. 16).
[Figure 6] Figure 6 shows cell proliferation curves of cells into which immortalizing factors (No. 1, No. 2, No. 4, No. 12, and No. 16) are introduced (up-arrow: morphological (transmitted light) image and fluorescent image point (Figure 5), *: telomere analysis point (Figures 11A and 11B), downward pointing triangle: chromosome analysis point).
[Figure 7] Figure 7 shows cell proliferation curves of cells into which three factors (Bmi-1/hTERT/SV40T) are introduced (NC: no introduction of the immortalizing genes, up-arrow: morphological (transmitted light) image and fluorescent image point (Figure 8), doubleheaded arrow: morphological (transmitted light) image point (Figure 9), *: telomere analysis point (Figure 11B), downward pointing triangle: chromosome analysis point (Figure 10)).
[Figure 8] Figure 8 shows transmitted light images and fluorescent (OFP and GFP) images of cells into which three factors (Bmi-1/hTERT/SV40T) are introduced.
[Figure 9] Figure 9 shows morphological (transmitted light) images of cells into which three factors (Bmi-1/hTERT/SV40T) are introduced (the temperature was maintained at 35°C or changed from 35°C to 37°C).
[Figure 10] Figure 10 shows chromosome analysis results (normal karyotype) obtained after 90 passage culture days of cells into which three factors (Bmi-1/hTERT/SV40T) are introduced (chromosome analysis point (downward pointing triangle) in Figure 7).
[Figure 11] Figure 11 shows telomere quantification analysis results in long-term culture samples of cells into which immortalizing factors (No. 1, No. 2, No. 4, No. 12, and No. 16) are introduced (A. the influence of a combination of immortalizing factors on telomere (Day 0, Day 40: telomere analysis point (*) in Figure 6), B. change in the amount of telomere between no removal and removal of factors after introduction of three factors (Bmi-1/hTERT/SV40T) (Day 0, Day 40: telomere analysis point (*) in Figure 6, Day 85, Day 98: telomere analysis point (^{∗}) in Figure 7).
[Figure 12] Figure 12 shows photographs adipocytes differentiated from cultured early MSCs and immortalized MSCs.
[Figure 13] Figure 13 shows photographs of osteoblasts differentiated from cultured early MSCs and immortalized MSCs.
[Figure 14] Figure 14 shows photographs of neurons differentiated from cultured early MSCs and immortalized MSCs.
[Figure 15] Figure 15 shows photographs of chondrocytes differentiated from cultured early MSCs and immortalized MSCs.
[Figure 16] Figure 16 shows cloning test results 1 about an immortalized cell population (2 weeks after introduction of immortalizing gene) (numeral in each well: the number of formed clones).
[Figure 17] Figure 17 shows cloning test results 2 about an immortalized cell population (4 months after introduction of immortalizing gene) (numeral in each well: the number of formed clones).
[Figure 18] Figure 18 shows a cell proliferation curve of a cloned immortalized cell (clone A10) (downward pointing triangle: chromosome analysis point).
[Figure 19] Figure 19 shows chromosome analysis results about a cloned immortalized cell (clone A10) (Day 80: chromosome analysis point (downward pointing triangle) in Figure 18).
[Figure 20] Figure 20 shows the differentiation potential of cloned immortalized cells (adipocyte differentiation, neuron differentiation, and osteoblast differentiation).
[Figure 21] Figure 21 shows cryopreservation points of MSCs used in a single-cell cloning test (early MSC: Day 9, intermediate MSC: Day 24, late MSC: Day 49) and induction points of immortalization by SeV vector infection (early MSC: Day 21, intermediate MSC: Day 36, late MSC: Day 61).
[Figure 22] Figure 22 shows photographs of SeV vector-infected cells (early MSC, intermediate MSC, and late MSC) and SeV vector-non-infected MSCs (control) before single-cell cloning (immediately before inoculation to a 96-well plate).
[Figure 23-1] Figure 23-1 shows single-cell cloning test results about cell populations of SeV vector-non-infected MSCs (control) and SeV vector-infected cells (early MSC) (numeral in each well: the number of formed clones, the number of culture days: 2 weeks).
[Figure 23-2] Figure 23-2 shows single-cell cloning test results about cell populations of SeV vector-infected cells (intermediate MSC and late MSC) (numeral in each well: the number of formed clones, the number of culture days: 2 weeks).
[Figure 24] Figure 24 shows SeV vector infection test results about cells that arrested their cell division (Day 90) (respective photographs of cells immediately before SeV vector infection, SeV vector-non-infected cells (control cells), and SeV vector-infected cells (after 2 weeks from the date of infection)).
[Figure 25] Figure 25 shows single-cell cloning test results about division-arrested cells that started re-proliferation by SeV vector infection (Day 90) (numeral in each well: the number of formed clones, the number of culture days: 2 weeks).
[Figure 26] Figure 26 shows cell proliferation curves of fat tissue-derived human MSCs (non-infected cell line and SeV vector-infected cell line) in serum-free culture.
[Figure 27] Figure 27 shows cell proliferation curves of bone marrow-derived human MSCs (non-infected cell line and SeV vector-infected cell line) in serum-free culture.
[Figure 28] Figure 28 shows transmitted light images and fluorescent (OFP and GFP) images of a SeV vector-infected cell line of fat tissue-derived human MSCs (hMSC-AT) and a SeV vector-infected cell line of bone marrow-derived human MSCs (hMSC-BM) cultured in a serum-free manner (hMSC-AT: 16 and 58 days from the date of infection, hMSC-BM: 24 and 50 days from the date of infection).
[Figure 29] Figure 29 shows cell proliferation curves of rat MSCs (non-infected cell line and SeV vector-infected cell lines #1 and #2).
[Figure 30] Figure 30 shows transmitted light images and fluorescent (OFP and GFP) images of a SeV vector-infected cell line of immortalized rat MSCs (2, 9, and 58 days from the date of infection).
[Figure 31] Figure 31 shows cell proliferation curves of HFL1 (non-infected cell lines #1 and #2, SeV vector-infected cell lines #1 and #2 cultured at 35°C, and SeV vector-infected cell lines #1 and #2 cultured at 37°C).
[Figure 32] Figure 32 shows transmitted light images and fluorescent (OFP and GFP) images of immortalized HFL1 [culture at 35°C (10, 34, and 255 days from the date of infection), culture at 35°C + 37°C (34 + 221 days from the date of infection)].
[Figure 33] Figure 33 shows cell proliferation curves of HUVEC (non-infected cell line #1, SeV vector-infected cell lines #1 and #2 cultured at 35°C, and SeV vector-infected cell lines #1 and #2 cultured at 37°C).
[Figure 34] Figure 34 shows transmitted light images and fluorescent (OFP and GFP) images of immortalized HUVEC [culture at 35°C (7, 36, and 72 days from the date of infection), culture at 35°C + 37°C (35 + 37 days from the date of infection)].
[Figure 35] Figure 35 is a photograph showing results of FISH analysis of immortalized MSCs by introduction of a multi-growth factor-inserted human artificial chromosome vector (a DAPI staining image of a cell indicated by the arrow is shown within the right lower frame).
[Figure 36] Figure 36 shows results of a healing effect test of a bowel inflammation model by the transplantation of immortalized MSCs.

### Description of Embodiments

### 1. Method for producing reversibly immortalized cell

The present invention provides a method for producing a reversibly immortalized cell. The method comprises the steps of (1) introducing chromosomally non-integrated RNA virus vector loaded with an immortalizing gene into a mammalian cell so that the immortalizing gene is expressed in the cell; and (2) culturing the cell obtained in the step (1) for proliferation.

### (Cell)

In the present invention, the "cell" refers to every somatic cell other than germline cells (eggs and sperms, oocytes, ES cells, etc.) and totipotent cells (iPS cells). The "somatic cell" may be any of primary cultured cells, passaged cells, and established cell lines. The somatic cell may be naturally derived or may be artificially prepared by the differentiation of iPS cells or the like. Specific examples of the somatic cell include differentiated cells such as cells that form tissues (adipocytes, fibroblasts, neurons, skin cells, blood cells, muscle cells, osteoblasts, chondrocytes, hepatocytes, pancreatic cells, renal cells, myocardial cells, brain cells, pneumocytes, splenocytes, adrenal cells, gingival cells, and periodontal ligament cells), and their progenitor cells, cells of the immune system (B cells, T cells, monocytic cells, etc.), and somatic stem cells [mesenchymal stem cells (fat-derived stem cells, bone marrow-derived stem cells, umbilical cord blood-derived stem cells, placenta-derived stem cells, etc.), hematopoietic stem cells, neural stem cells, epidermal stem cells, intestinal epithelial stem cells, dental pulp stem cells, periodontal ligament stem cells, etc.]. The origin of the cell is not particularly limited as long as the origin is a mammal. Examples thereof include humans, mice, rats, guinea pigs, hamsters, rabbits, dogs, cats, pigs, horses, and bovines.

### (Reversibly immortalized cell)

Unlike primary cultured cells or cells cultured under usual culture conditions, the "immortalized cell" refers to a cell that does not arrest its proliferation even by repeated cell division, i.e., a cell having infinite proliferative potential. The "immortalized cell" according to the present invention is a cell capable of proliferating infinitely by introduction of a predetermined immortalizing gene, and refers to a cell that does not reduce its infinite proliferative potential even when repetitively passaged and cultured. The "immortalized cell" according to the present invention differs in proliferation rate or proliferation period depending on the origin of the cell or culture conditions, and as a result of passage culture, is capable of continuing exponential proliferation for 20 days or longer, preferably 60 days or longer, more preferably 80 days or longer, even after the time when untreated cells reduce or arrest their proliferation under the same culture conditions. The immortalized cell of the present invention encompasses any of cell populations capable of proliferating infinitely as described above, and immortalized cell lines cloned from the cell populations. The "reversible immortalization" refers to the arrest or attenuation of cell proliferation by the removal of an immortalizing gene after the immortalizing gene is introduced into a cell and thereby the cell is rendered capable of proliferating infinitely.

The "immortalization" refers to the imparting of continuous cell division potential and proliferative potential by canceling limitations on the number of cell division rounds of early cells or cellular senescence. Specifically, the immortalization refers to a state that permits usually 5 or more passages, preferably 7 or more passages, 8 or more passages, 9 or more passages, 10 or more passages, 12 or more passages, 15 or more passages, 20 or more passages under standard cell culture conditions. Confluent cells at a passage number of 0 can be amplified and cultured by a passage operation according to an approach known to those skilled in the art. A cell obtained by one passage operation refers to a cell at a "passage number of 1 (or second generation)" and can be expressed as a "passage number of 2, 3, 4 ... n (n (integer) represents a passage number) ((n + 1)th generation)" in response to the number of passage operations. The step of freezing cells may be included between passage operations.

### (Immortalizing gene)

The immortalized cell of the present invention is prepared by introducing a predetermined immortalizing gene into a cell using a chromosomally non-integrated RNA virus vector. In this context, the "immortalizing gene" refers to a gene that immortalizes a cell so as to acquire infinite proliferative potential, and does not induce cell death. The immortalizing gene is an exogenous gene and means an immortalizing gene newly transferred from the outside of a cell. The immortalizing gene may be a non-human derived immortalizing gene or may be an immortalizing gene engineered in a form that can be expressed in a target cell. In the present invention, one or two or more gene(s) selected from the group consisting of Bmi-1 gene, TERT gene, and SV40T gene can be used as the immortalizing gene. A combination of two of the genes is preferred, and a combination of three of the genes is more preferred. In the case of using one of the genes, TERT gene is preferred. In the case of combining two or more of the genes, preferred examples of the combination include a combination of Bmi-1 gene and TERT gene, and a combination of TERT gene and SV40T gene.

In the present invention, the "gene" encompasses not only structural genes which define the primary structure of a protein, but regions, such as a promoter and an operator, on a nucleic acid having a control function, unless otherwise specified. Thus, the "gene" according to the present invention refers to a regulatory region, a coding region, an exon, and an intron without distinction, unless otherwise specified.

"Bmi-1 (B lymphoma Mo-MLV insertion region 1 homolog) gene" is a gene encoding a protein that functions as a polycomb repressive complex 1 (PRC1) which has two nuclear localization signals and is localized in the cytoplasm or the nucleus. Bmi-1 is involved as a PRC1 in the expression control of various genes including Hox gene by the control of chromatin remodeling or histone modification. Bmi-1 is known to have an effect of controlling cell proliferation by suppressing the expression of p16 or p19Arf involved in cell cycles, and to play an important role in maintaining autonomous replication by participating in the cell division of hematopoietic stem cells or neural stem cells.

"TERT (telomerase reverse transcriptase) gene" is a gene encoding telomere reverse transcriptase (TERT). The telomere reverse transcriptase (TERT) constitutes telomerase, an enzyme that elongates a specific repeat sequence at the chromosome end (telomere) of a eukaryote from telomerase RNA (TR) or telomerase RNA component (TERC) and other control subunits. Cells have a telomere length surveillance mechanism, and cellular senescence is caused by the shortening of telomere. TERT is known to play a role in maintaining a telomere length.

"SV40T (simian virus 40 large T antigen) gene" is a gene encoding a simian virus 40 large T antigen. The genome of SV40 (simian vacuolating virus 40) is divided into an early region which is expressed immediately after infection, a late region which is expressed during the replication of the virus genome after infection, and a regulatory region containing transcriptional control and replication origins. The early region encodes a large T antigen which is involved in the initiation of virus genome replication and the inactivation of tumor suppressor gene products p53 and pRB, and a small t antigen which binds to and inhibits a protein dephosphorylating enzyme PP2A.

Specific examples of the Bmi-1 gene used in the present invention include mouse BMI1 gene (SEQ ID NO: 1). Specific examples of the TERT gene include human TERT gene (SEQ ID NO: 2). Specific examples of the SV40T gene include SV40 large T antigen gene (SEQ ID NO: 3). The Bmi-1 gene, the TERT gene, and the SV40T gene may also be their transcriptional mutants, splicing mutants, and orthologs thereof.

The Bmi-1 gene, the TERT gene, and the SV40T gene described above may be genes consisting of nucleotide sequences having 80% or higher, preferably 90% or higher, more preferably 95% or higher sequence identity to the nucleotide sequences of SEQ ID NOs: 1, 2, and 3, respectively, or genes derived from the nucleotide sequences of SEQ ID NOs: 1, 2, and 3, respectively, by the substitution, insertion, addition and/or deletion of several (e.g., 1 to 30, preferably 1 to 20, more preferably 1 to 10, further preferably 1 to 5, particularly preferably 1 to 3) bases as long as the genes have a function and activity equivalent thereto. Such homolog genes are also encompassed in the immortalizing gene according to the present invention. The Bmi-1 gene, the TERT gene, and the SV40T gene may be genes artificially modified such that products of the genes are expressed as fusion proteins with other proteins or peptides as long as the genes have a function and activity equivalent thereto.

### (Chromosomally non-integrated RNA virus vector)

In the present invention, a "chromosomally non-integrated RNA virus vector" is used as a vector for the introduction of the immortalizing gene into a cell for the expression. In the present invention, the virus vector means a vector that has a genomic nucleic acid derived from the virus, and enables the gene to be expressed by the incorporation of the transgene in the nucleic acid. The "chromosomally non-integrated RNA virus vector" is a virus vector that is derived from the virus and enables the gene to be introduced into a target cell, and refers to a risk-free vehicle that integrates the introduced gene into the chromosome (nucleus-derived chromosome) of a host.

Examples of the chromosomally non-integrated RNA virus vector used in the present invention include negative-strand RNA virus vectors. The "negative-strand RNA virus vector" refers to a vector of a virus containing negative-strand (antisense strand complementary to a sense strand encoding a virus protein) RNA as the genome. The negative-strand RNA virus used in the present invention is particularly preferably a single-stranded negative-strand RNA virus (also called non-segmented negative-strand RNA virus). The "single-stranded negative-strand RNA virus" refers to a virus having single-stranded negative-strand RNA in the genome and includes, for example, viruses that belong to families such as the family *Paramyxoviridae* (including the genera *Paramyxovirus, Morbillivirus, Rubulavirus,* and *Pneumovirus,* etc.), the family *Rhabdoviridae* (including the genera *Vesiculovirus, Lyssavirus,* and *Ephemerovirus,* etc.), and the family *Filoviridae.*

Examples of the negative-strand RNA virus that can be used in the present invention include Sendai virus, Newcastle disease virus, mumps virus, measles virus, RS virus (respiratory syncytial virus), rinderpest virus, distemper virus, simian parainfluenza virus (SV5), and human parainfluenza virus types 1, 2, and 3 of the family *Paramyxoviridae,* influenza virus of the family *Orthomyxoviridae,* and vesicular stomatitis virus and rabies virus of the family Rhabdoviridae. Sendai virus is preferred.

### (Sendai virus vector: SeV vector)

In a preferred aspect of the present invention, a Sendai virus (SeV) vector is used as the chromosomally non-integrated RNA vector. The respective immortalizing factor genes, i.e., the Bmi-1 gene, the TERT gene, and the SV40T gene, may be inserted in separate SeV vectors or may be inserted together in a single SeV vector.

The Sendai virus is a virus of the genus *Respirovirus* of the family *Paramyxoviridae* and contains single negative-strand (antisense strand of a sense strand encoding a virus protein) RNA as the genome.

The SeV vector has features such as: (i) to have exceedingly high gene introduction and expression efficiency into various mammalian cells including human cells; (ii) to have no risk of structural change of the chromosome because this vector is a chromosomally non-integrated virus vector and does not integrate the transgene into the chromosome of a host (the transgene is expressed in the cytoplasm); (iii) to be not a human pathogenic virus; (iv) to permit adjustment of a gene expression level and coexpression of a plurality of genes by change of an insertion position in the vector; and (v) to be removable from the transgenic cells after objective achievement.

The genome of the Sendai virus contains NP (nucleocapsid) gene, P (phospho) gene, M (matrix) gene, F (fusion) gene, HN (hemagglutinin/neuraminidase) gene, and L (large) gene in order from the 3' end toward the 5' end. The Sendai virus can function sufficiently as a vector, can replicate its genome in a cell, and enables the gene loaded therein to be expressed, as long as the virus has the NP gene, the P gene, and the L gene among those genes. Since the Sendai virus has negative-strand RNA in the genome, the 3' side and the 5' side of the genome correspond to upstream and downstream sides, respectively, on the contrary to normal.

Any of natural strains, wild strains, mutant strains, and commercially available products can be used as the SeV vector according to the present invention. The virus may be a virus structurally similar to a naturally isolated virus or may be a virus artificially engineered by gene recombination as long as the function of interest can be achieved. The virus may have, for example, a mutation or deficiency in any gene of the wild-type virus. Specifically, a non-transmissible vector that lacks the F gene in the genome and is free of the formation of infective particles from transgenic cells (ΔF), or a vector that lacks the F gene and further lacks the M and/or HN gene or further has a mutation in the M and/or HN gene (e.g., temperature sensitivity mutation) is suitably used in the present invention. Also, for example, a vector that lacks the F gene, further lacks the M or HN gene, and also further has a mutation in the remaining M and/or HN gene (e.g., temperature sensitivity mutation) is suitably used in the present invention (see JP Patent No. 5763340).

The SeV vector for use in the method of the present invention is preferably temperature-sensitive. The "temperature sensitivity" means that activity is significantly reduced at a usual cell culture temperature (e.g., 37 to 38°C) compared with a low temperature (e.g., 30 to 36°C). For example, a mutation such as TS7 (Y942H/L1361C/L1558I mutation of the L protein), TS12 (D433A/R434A/K437A mutation of the P protein), TS13 (D433A/R434A/K437A mutation of the P protein and L1558I mutation of the L protein), TS14 (D433A/R434A/K437A mutation of the P protein and L1361C of the L protein), or TS15 (D433A/R434A/K437A mutation of the P protein and L1361C/L1558I of L protein) of the Sendai virus is temperature sensitivity mutation and can be suitably used in the present invention. Such a mutation is preferably further introduced to the SeV vector that lacks the F gene. For these SeV vectors, see JP Patent No. 5763340, WO2015/046229, and the like.

The SeV vector according to the present invention includes infective virus particles as well as a virus core, a complex of the virus genome and a virus protein, or a complex that has non-infective virus particles and has the ability to express a loaded gene by the introduction into cells. For example, a ribonucleoprotein (virus core moiety) composed of the Sendai virus genome and Sendai virus proteins (NP, P, and L proteins) that bind thereto enables a transgene to be expressed in cells by the introduction into the cells. The introduction into cells can be appropriately performed using a transfection reagent or the like. Thus, such a ribonucleoprotein (RNP) is also encompassed in the SeV vector according to the present invention.

### (Construction of SeV vector loaded with immortalizing gene)

The position at which the immortalizing gene (Bmi-1 gene, TERT gene, and SV40T gene) is incorporated is not particularly limited. In the case of inserting the respective immortalizing genes to a plurality of separate vectors, the Bmi-1 gene is preferably inserted upstream of the NP gene; the TERT gene is preferably inserted between the P gene and the M gene; and the SV40T gene is preferably inserted upstream of the NP gene. Two or more (Bmi-1 and TERT, TERT and SV40T, or Bmi-1, TERT, and SV40T) genes may be inserted in a single vector.

The SeV vector loaded with the immortalizing gene may be prepared into a kit. The kit can contain, for example, a medium and a container for cell culture, and an instruction that states a method for using the kit.

### (Introduction of immortalizing gene into cell)

The SeV vector loaded with the immortalizing gene obtained as described above is introduced into a somatic cell, by adding the vector (Sendai virus particle) to a medium of the cell and infecting the cell with the virus. The dose of the vector differs depending on the type of the cell, a cell density, and the amount of the medium and can therefore be determined by investigating MOI that attains infection efficiency of 100% in advance on the basis of each cell used.

Alternatively, the SeV vector in the form of RNP can be used in the introduction into the cell by an approach, for example, electroporation, lipofection, or microinjection.

### (Culture of cell into which immortalizing genes are introduced)

In the present invention, a method for culturing the cell into which the immortalizing genes are introduced can be performed in accordance with a method and conditions for culturing usual mammalian somatic cells. The medium for use in culture is not particularly limited, and a medium that is generally used for the maintenance culture or expansion culture of cells and is suitable for viral infection can be used. Any of commercially available media and self-made medium can be used. Examples thereof include basal media containing components necessary for cell survival and proliferation (inorganic salts, carbohydrates, hormones, essential amino acids, non-essential amino acids, vitamins, and fatty acids), specifically, Dulbecco's Modified Eagle's Medium (D-MEM) medium, Dulbecco's Modified Eagle's Medium:Nutient Mixture F-12 (D-MEM/F-12) medium, Glasgow MEM (G-MEM) medium, Basal Medium Eagle (BME) medium, Minimum Essential Medium (MEM) medium, Eagle's minimal essential medium (EMEM) medium, Iscove's Modified Dulbecco's Medium (IMDM) medium, RPMI 1640 medium, Medium 199 medium, aMEM medium, Ham medium, Fischer medium, and mixed media thereof. The medium may optionally contain a growth factor (FGF, EGF, etc.), interleukins, insulin, transferrin, heparin, heparan sulfate, collagen, fibronectin, progesterone, selenite, B27-supplement, N2-supplement, an antibiotic (penicillin, streptomycin, etc.), and the like. The medium may be a serum-containing medium or may be a serum-free medium. From the viewpoint of preventing contamination by heterozoic components, it is preferred to contain no serum or to use serum derived from the same animal as that of the cell to be cultured. Alternatively, a serum replacement such as albumin may be used.

Examples of the culture method include, but are not limited to, three-dimensional culture under non-adherent conditions, for example, suspension culture (e.g., dispersion culture and aggregate suspension culture), and two-dimensional culture under adherent conditions, for example, plate culture, and combined culture of three-dimensional culture and two-dimensional culture. The culture vessel for use in cell culture is not particularly limited as long as the culture vessel permits cell culture. Examples thereof include flasks, petri dishes, dishes, plates, chamber slides, tubes, trays, culture bags, and roller bottles. The culture vessel may be non-cell-adherent or may be cell-adherent and is appropriately selected depending on a purpose. The cell-adherent culture vessel may be treated, for use, with a substrate for cell support using extracellular matrix or the like for the purpose of improving cell adherence. Examples of the substrate for cell support include collagen, gelatin, poly-L-lysine, poly-D-lysine, laminin, and fibronectin.

The culture temperature is 30°C to 36°C, preferably 32°C to 35°C, more preferably 33 to 35°C. The culture is performed in an atmosphere of CO₂-containing air, for example, at a CO₂ concentration of 2% to 5%. The culture temperature for removing the immortalizing gene is 37°C to 38°C, preferably 37°C to 37.5°C.

### 2. Induction of differentiation

According to the present invention, the immortalized cell prepared as described above can be cultured in a medium for induction of differentiation to induce differentiation into a particular tissue cell. For example, when the immortalized cell is a mesenchymal stem cell, for example, adipocyte, osteoblast, neuron, or chondrocyte differentiation can be achieved.

The composition of a medium, a differentiation-inducing factor, a culture method, a passage method, and the like for inducing the differentiation of the immortalized cell according to the present invention into the cell of interest can be appropriately set from a well-known or routine technique.

The medium for induction of differentiation can be appropriately selected depending on the type of the cell of interest in the induction of differentiation. Media for induction of differentiation into various tissues (media supplemented with at least one differentiation-inducing or -promoting factor according to the cell of interest in the induction of differentiation) are commercially available, and these commercially available media may be used. For example, a commercially available adipocyte induction medium or a commercially available medium for animal cells containing insulin, dexamethasone, indomethacin, 3-isobutyl-1-methylxanthine, troglitazone, biotin, and the like can be used as a medium for inducing the adipocyte differentiation of the immortalized cell according to the present invention. Examples of the commercially available medium include Mesenchymal Stem Cell Adipogenic Differentiation Medium 2 (manufactured by PromoCell GmbH), and Human Mesenchymal Stem Cell Adipogenic Differentiation Medium Bullet Kit 8 (manufactured by Lonza Group AG). A commercially available osteoblast induction medium or a commercially available medium for animal cells containing dexamethasone, ascorbic acid, β-glycerophosphoric acid, hydrocortisone, BMP4, BMP2, and the like can be used as a medium for inducing the osteoblast differentiation of the cell according to the present invention. Examples of the commercially available medium include Mesenchymal Stem Cell Osteogenic Differentiation Medium (manufactured by PromoCell GmbH), and Human Mesenchymal Stem Cell Osteogenic Differentiation Medium Bullet Kit (manufactured by Lonza Group AG). A commercially available neuron culture medium or neuron differentiation medium (e.g., Mesenchymal Stem Cell Neurogenic Differentiation Medium (manufactured by PromoCell GmbH)) can be used as a medium for inducing the neuron differentiation of the immortalized cell according to the present invention. The neuron culture medium or the medium for induction of neuron differentiation preferably contains a neuron-inducing factor (e.g., brain-derived nerve growth factor (BDNF) and fibroblast growth factor (FGF)). A commercially available chondrocyte induction medium or a commercially available medium for animal cells containing dexamethasone, ascorbic acid, and TGF-β3 can be used as a medium for inducing the chondrocyte differentiation of the immortalized cell according to the present invention. Examples of the commercially available medium include Mesenchymal Stem Cell Chondrogenic Differentiation Medium (manufactured by PromoCell GmbH), and Human Mesenchymal Stem Cell Chondrogenic Differentiation Medium Bullet Kit (manufactured by Lonza Group AG).

The culture conditions for induction of differentiation are similar to those for culturing usual stem cells. The culture period for induction of differentiation is not particularly limited and is generally 5 days to 20 days, preferably 7 days to 18 days.

Whether or not the differentiation of the stem cell into the cell of interest has been induced can be confirmed by examining the expression of a marker specific for each differentiated cell. For example, the adipocyte differentiation can be confirmed by oil red 0 staining; the osteoblast differentiation can be confirmed by alkaline phosphatase staining; the neuron differentiation can be confirmed by NeuroFluor NeuO staining; and the chondrocyte differentiation can be confirmed by Alcian blue staining.

The immortalized cell obtainable by the present invention or a tissue cell induced by the differentiation of the immortalized (stem) cell can be used in, for example, cell transplantation to a disease or injury site, and can be used as a product for regenerative medicine. Examples of the product for regenerative medicine include cultured skin, cultured cartilage, cultured corneal epithelium, and various cell sheets (e.g., epidermal cell sheets, fibroblast sheets, corneal endothelial cell sheets, myocardial cell sheets, osteoblast sheets, myoblast sheets, neuron sheets, chondrocyte sheets, hepatocyte sheets, pancreatic islet cell sheets, and periodontal ligament cell sheets).

### Examples

Hereinafter, the present invention will be described in more detail with reference to Examples. However, the present invention is not limited by these Examples.

In the following Examples, a serum-containing medium for culturing human mesenchymal stem cells (MSCs) was prepared with composition of D-MEM (low glucose), 20% FBS, 0.01 mol/L Hepes, 100 units/ml penicillin, 100 µg/ml streptomycin, and 20 ng/ml bFGF. Since bFGF has a very short half-life, Gibco Heat Stable Recombinant Human bFGF (Thermo Fisher Scientific Inc) excellent in stability was used. MSCs were cultured in the medium in a 5% CO₂ incubator. Human MSCs used in experiments were any of bone marrow-derived, fat tissue-derived, umbilical cord blood-derived, and umbilical cord matrix-derived MSCs.

### (Example 1) Preparation of SeV vector loaded with immortalizing gene and study on infection conditions

### (1) Preparation of SeV vector loaded with immortalizing gene

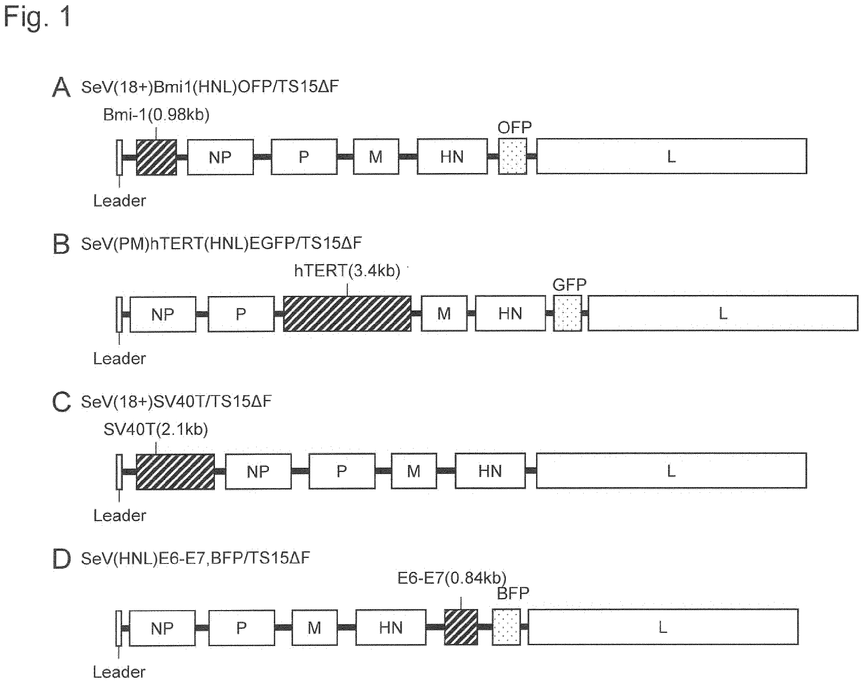

General-purpose immortalizing factors Bmi-1 (B lymphoma Mo-MLV insertion region 1 homolog), hTERT (human telomerase reverse transcriptase), SV40T (simian virus 40 large T antigen), and E6/E7 (human papillomavirus 16 E6 protein and E7 protein) were selected, and the Bmi-1 gene (SEQ ID NO: 1), the hTERT gene (SEQ ID NO: 2), the SV40T gene (SEQ ID NO: 3), and the E6/E7 gene (SEQ ID NO: 4) were each incorporated in a SeV vector as a chromosomally non-integrated RNA virus vector. The loading of the SeV vector with each immortalizing gene and the production of the vector were commissioned to ID Pharma Co., Ltd. (headquarter: Tsukuba-shi, Ibaraki, Japan). Three types of vectors among these four types of vectors were also loaded with fluorescent dye genes of ORF (red), GFP (green), and BFP (blue), respectively, at the same time therewith such that the presence or absence of gene expression (vector genome) was able to be easily confirmed under a microscope. Figure 1 shows the structure of each vector. As for the position of the immortalizing gene to be loaded in the SeV vector, the Bmi-1 or SV40T gene was loaded most upstream in the vector genome because higher expression results in longer-term expression and upstream loading in the vector genome attains high expression for the SeV vector. The loading position of the hTERT or E6/E7 gene was changed to a downstream site due to reduction in vector production efficiency presumably caused by high expression.

The SeV vector used was a vector of TS15ΔF type modified such that the virus vector would disappear from cells by changing a culture temperature from 35°C to 37°C (Efficient generation of transgene-free human induced pluripotent stem cells (iPSCs) by temperature-sensitive Sendai virus vectors. Ban H, Nishishita N, Fusaki N, Tabata T, Saeki K, Shikamura M, Takada N, Inoue M, Hasegawa M, Kawamata S, Nishikawa S. Proc Natl Acad Sci USA. 2011 Aug 23; 108 (34): 14234-9).

### (2) Study on SeV vector infection conditions

For gene introduction of the SeV vector, infection efficiency varies largely depending on difference in cell type. Excessive infection may cause toxicity depending on cell type. Accordingly, MOI (multiplicity of infection: the number of vector particles per cell) for MSCs was first studied in order to determine MOI beforehand for the cell type used.

Commercially available bone marrow-derived mesenchymal stem cells (product name: Ultrahigh-Purity Human Mesenchymal Stem Cells (REC), PuREC Co., Ltd.) were inoculated at 5 × 10⁴ cells/well (70-80% confluent) to a 48-well plate (FALCON 353230) supplemented with a medium at 200 µl/well, and the four types of SeV vectors loaded with the immortalizing factor genes were added to these cells such that MOI of each vector was 1, 5, or 20 by infection with each vector alone or coinfection with a plurality of vectors, followed by overnight incubation. On the next day, the medium was replaced with a fresh one, and the medium was then replaced every two days. The cells were expanded to a 12-well plate (FALCON 353043) 5 days after vector infection and observed 9 days after infection.

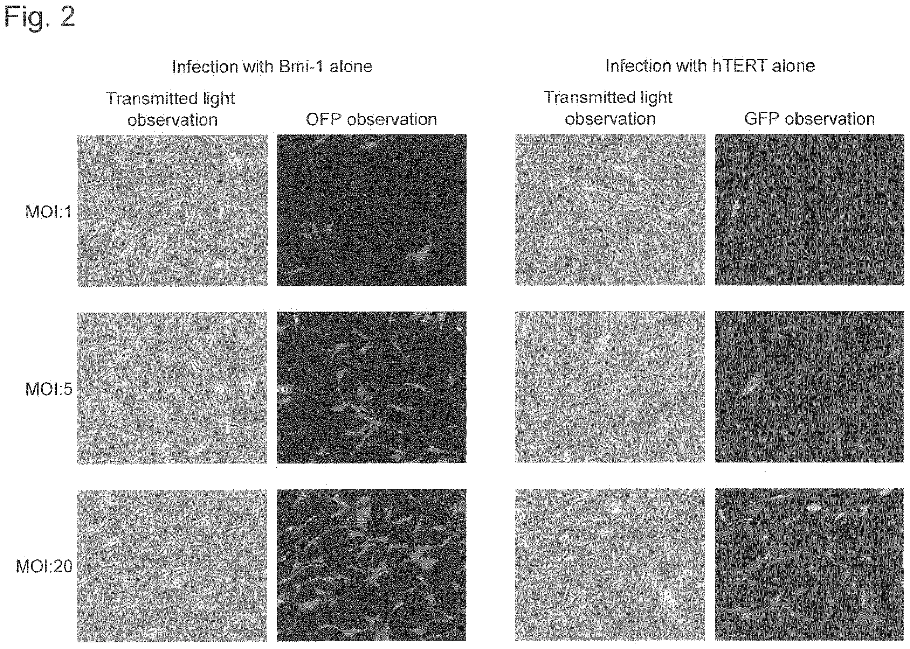

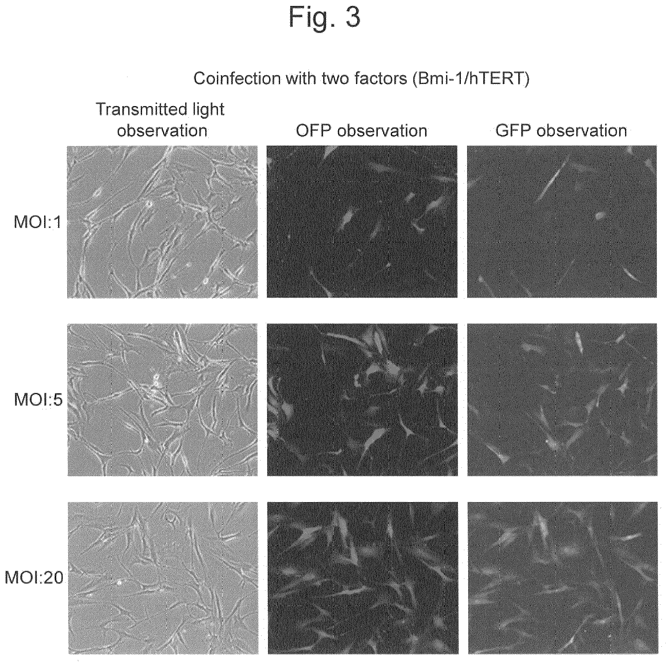

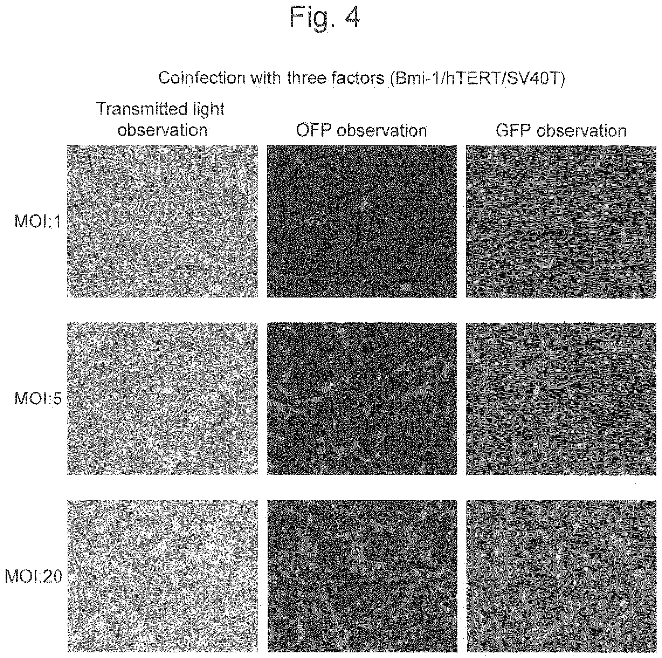

The results are shown in Figures 2 to 4. No cytotoxicity was found in any of the wells 9 days after SeV vector infection, and no abnormality was found at MOI: 20 (MOI of the SeV vector itself: 80). When the infection efficiency of the SeV vector was observed under an inverted fluorescence microscope (Nikon Corp.) through fluorescent dye expression, the fluorescent dye expression was found in most of the cells infected with the vector alone at MOI: 20 (Figure 2). Similar results were also obtained in coinfection with two factors Bmi-1 and hTERT (Figure 3). Similar results were also obtained in coinfection with three factors Bmi-1, hTERT, and SV40T (Figure 4). This suggested that infection at MOI: 20 permits gene introduction into almost 100% cells. Accordingly, MOI of the subsequent SeV vector infection of MSCs was determined as 20.

Coinfection with three vectors of Bmi-1, hTERT, and SV40T among the combinations of the SeV vectors studied was confirmed to evidently increase the number of cells at an infection ratio as high as MOI: 20, as compared with other combinations including an uninfected control (Figure 4). Actively dividing cells at MOI: 20 were smaller in size than cells found to exhibit no fluorescence at MOI: 1, and were positive for both the fluorescence of ORF and the fluorescence of GFP. In short, this promotion of cell proliferation was presumably because three immortalizing factors Bmi-1, hTERT, and SV40T worked.

### (Example 2) Selection of immortalizing factor

MSCs were infected with a SeV vector loaded with each of four immortalizing factor (Bmi-1, hTERT, SV40T, and E6/E7) genes alone, and cultured for a long period to select a factor necessary for the immortalization of MSCs.

Umbilical cord blood-derived MSCs (product name: Umbilical Cord-Derived Mesenchymal Stem Cells; Normal, Human (ATCC PCS-500-010)) were used in study, and the MSCs were infected (48-well plate, 3 × 10⁴ cells/well, MOI: 20) with one or two or more in combination of the SeV vectors loaded with the immortalizing factor genes (a total of 15 patterns). MSCs that were not infected with the SeV vectors were used as a negative control. As the MSCs proliferated, expansion culture was performed using 48 wells -> 12 wells -> 6 wells. After subsequent confluency (the adherent surface of the plate was covered 100% with cells), the number of cells was measured, and the proliferation rates of a total of 16 types of cells were measured by a passage while the cells were morphologically observed under a microscope.

The proliferation rates of the cells were measured over 80 days from introduction of immortalizing gene (the number of cells at the time of introduction: 3 × 10⁴ cells). Results of listing in the descending order of the number of cells on day 80 (No. 1 to No. 16) are shown in Table 1 below, together with the number of cells.

**[Table 1]**

| No. | Presence or absence of immortalizing factor (+or-) | | | | Day 59 | Day 80 |
|---|---|---|---|---|---|---|
| | Bmi-1 | hTERT | SV40T | E6E7 | Rating in cell observation | Approximate number of cells |
| 1 | + | + | + | - | ○ | 1.7.E+19 |
| 2 | + | + | - | - | ○ | 3.1.E+18 |
| 3 | + | + | + | + | ○ | 2.0.E+18 |
| 4 | - | + | + | - | ○ | 1.3.E+18 |
| 5 | + | + | - | + | ○ | 6.3.E+17 |
| 6 | + | - | + | - | × | 4.4.E+17 |
| 7 | - | + | + | + | ○ | 1.9.E+17 |
| 8 | - | + | - | + | ○ | 1.1.E+17 |
| 9 | + | - | + | + | ○ | 8.0.E+16 |
| 10 | + | - | - | + | × | 9.2.E+15 |
| 11 | - | - | + | - | × | 6.0.E+15 |
| 12 | - | + | - | - | ○ | 1.4.E+15 |
| 13 | - | - | + | + | × | 1.1.E+15 |
| 14 | - | - | - | + | × | 4.8.E+14 |
| 15 | + | - | - | - | × | 1.4.E+13 |
| 16 | - | - | - | - | × | 1.2.E+12 |

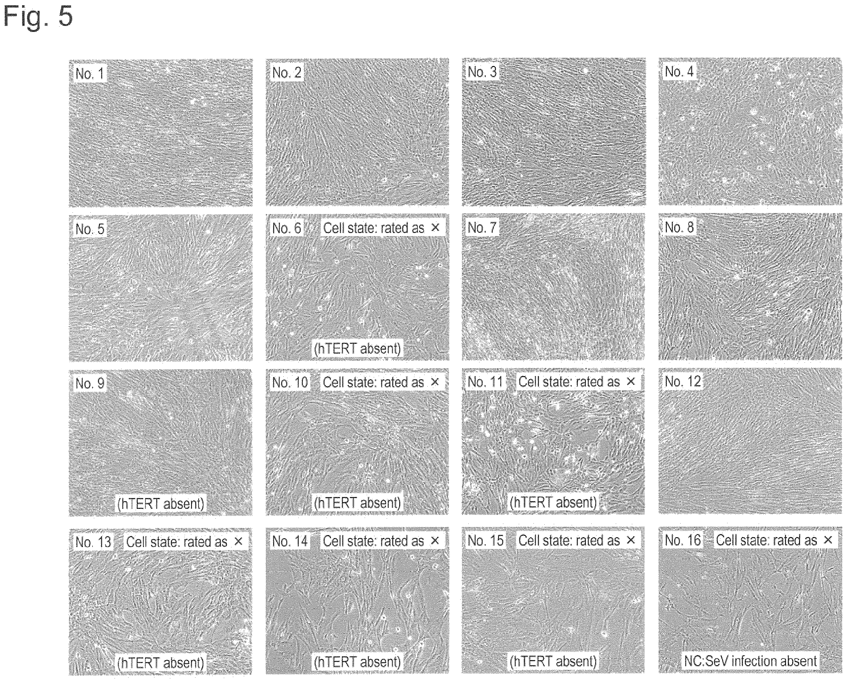

The states of the cells were observed on approximately 2 months (day 59) when the cell proliferation of the negative control was arrested. Uniformity in the sizes of the cells and the degrees of dead cells detached from the culture plate were rated on two scales (Figure 5). The rating results are also shown in Table 1 (circle: uniform size of cells and less detachment of cells, X-mark: non-uniform size of cells and more detachment of cells; the presence or absence of the immortalizing factor is indicated by + or -).

As shown in Table 1 and Figure 5, the morphological abnormality of the cells was found in all the combinations (including the negative control) without the hTERT factor, suggesting that hTERT is essential for cell immortalization using the SeV vector. As a result of comparing the numbers of cells and the combinations of the immortalizing factors, E6/E7 among the four immortalizing factors was found to rarely have a cell proliferative effect.

From the results described above, combinations of the immortalizing factors suitable for immortalization using the SeV vector were extracted from Table 1 and summarized in Table 2 (No. 16 is shown for comparison as a negative control).

**[Table 2]**

| No. | Bmi-1 | hTERT | SV40T | Day 80 | Day 59 |
|---|---|---|---|---|---|
| | | | | Approximate number of cells | Rating in cell observation |
| 1 | + | + | + | 1.7.E+19 | ○ |
| 2 | + | + | - | 3.1.E+18 | ○ |
| 4 | - | + | + | 1.3.E+18 | ○ |
| 12 | - | + | - | 1.4.E+15 | ○ |
| 16 | - | - | - | 1.2.E+12 | × |

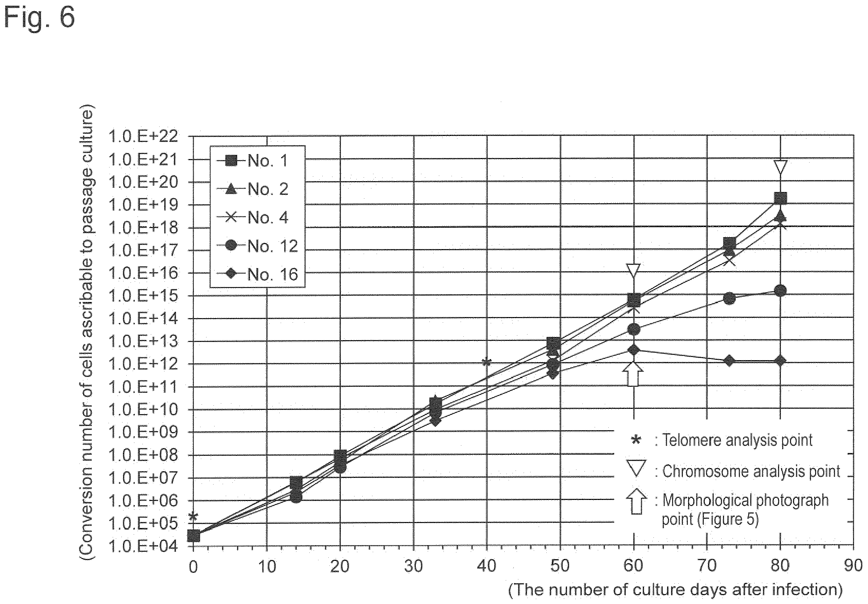

Figure 6 shows the proliferation curves of the cells. The negative control (No. 16) arrested its proliferation approximately 2 months (66 days) later, whereas the cells into which immortalizing factors are introduced (Nos. 1, 2, 4, and 12) had extended proliferation. The proliferation rate of the cells into which the hTERT gene alone is introduced (No. 12) was gradually decreased on 2 months or later, though their proliferation was extended. Thus, further proliferation was not able to be expected. By contrast, the cells into which Bmi-1 and hTERT, hTERT and SV40T, or Bmi-1, hTERT, and SV40T are introduced in combination (Nos. 1, 2, and 4) were observed to proliferate stably even on day 80 without slowing down a proliferation rate. Among them, the combination of three factors Bmi-1, hTERT, and SV40T exhibited the fastest proliferation rate.

Study was made on combinations of the immortalizing factors using human umbilical cord matrix-derived mesenchymal stem cells (product name: Human Mesenchymal Stem Cells from Umbilical Cord Matrix (hMSC-UC), PromoCell GmbH, product code: C-12971) instead of the umbilical cord blood-derived MSCs described above. As a result, the cells immortalized with Bmi-1, hTERT, and SV40T in combination had the best proliferation, as in the above, and also, the cells were homogeneous and had the best state when observed under a microscope.

### (Example 3) Analysis of cultured cell immortalized with SeV vectors loaded with three factor (Bmi-1/hTERT/SV40T) genes

### (1) Preparation of immortalized cell into which three factors (Bmi-1/hTERT/SV40T) are introduced

In the study of the MSCs derived from three types of different tissues, the SeV vectors loaded with three factor (Bmi-1/hTERT/SV40T) genes were confirmed to be most suitable for immortalization. MSCs immortalized with the vectors were analyzed for their properties. The cells used were umbilical cord blood-derived MSCs (product name: Umbilical Cord-Derived Mesenchymal Stem Cells; Normal, Human (ATCC PCS-500-010)) used in the selection of the immortalizing factors except that the cells were cultured for a longer period (by a little less than 1 month) than that for the selection. Gene introduction was performed with the SeV vectors loaded with three factor (Bmi-1/hTERT/SV40T) genes under the same conditions (MOI: 20) as in Example 2. MSCs that did not undergo gene introduction of the SeV vectors were also cultured as a negative control.

### (2) Comparison of proliferation rate depending on change of culture temperature

The immortalized cell line into which three factors (Bmi-1/hTERT/SV40T) are introduced was cultured in a CO₂ incubator of 35°C using a 6 cm dish, and the culture was continued for 75 days by a method of passaging 1/5 of the culture solution to a fresh 6 cm dish at the proliferation time when the adherent surface of the plate was covered approximately 80% with the cells.

The SeV vectors loaded with the immortalizing genes are temperature-sensitive vectors, and the SeV vector genome is capable of disappearing rapidly in cells by elevating a culture temperature from 35°C to 37°C (Efficient generation of transgene-free human induced pluripotent stem cells (iPSCs) by temperature-sensitive Sendai virus vectors. Ban H, Nishishita N, Fusaki N, Tabata T, Saeki K, Shikamura M, Takada N, Inoue M, Hasegawa M, Kawamata S, Nishikawa S. Proc Natl Acad Sci U S A. 2011 Aug 23; 108 (34): 14234-9). Accordingly, in order to confirm whether the SeV vector genome was also removable in MSCs, the temperature was changed in the culture of the immortalized cells into which three factors (Bmi-1/hTERT/SV40T) are introduced to examine change in the number of cells.

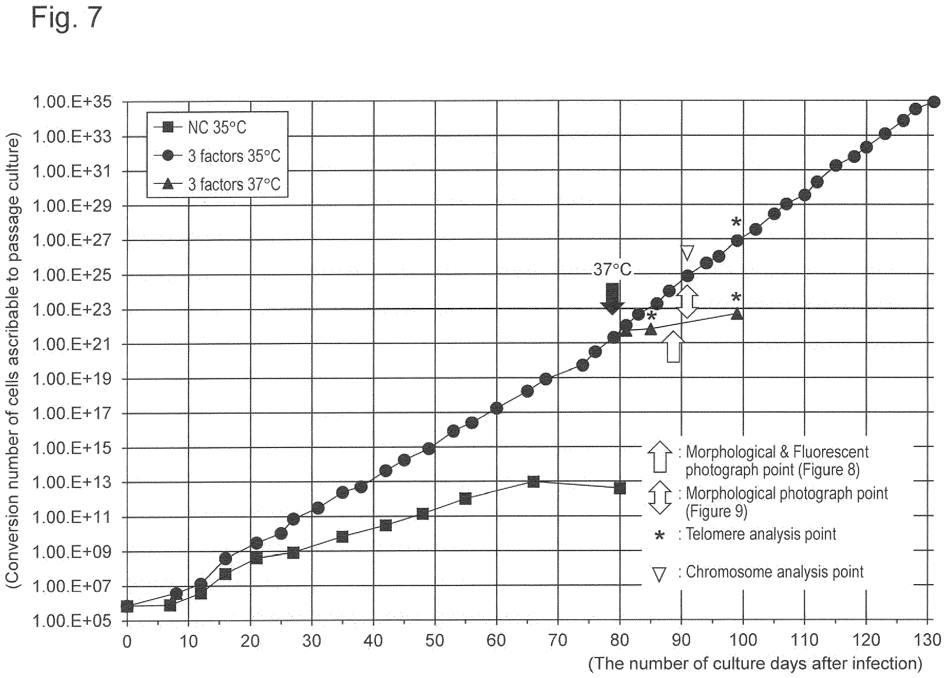

Immortalized cells of untreated MSCs at the time when proliferative potential was reduced (day 75) were used in the study of removal of the SeV vectors by temperature change. Two petri dishes in which the same numbers of cells were inoculated were prepared upon cell passage on day 78. One of the petri dishes was maintained at 35°C, while the culture temperature was elevated to 37°C for the other petri dish to compare both the proliferation patterns. The results are shown in Figure 7. In the cells cultured at 37°C, the SeV vectors were removed (the fluorescence disappeared), and proliferation was rapidly reduced. By contrast, the cells cultured at 35°C had no particular change and continued to proliferate actively even for more than 130 days after gene introduction (Figure 7).

### (3) Comparison of fluorescent protein expression and cell morphology depending on change of culture temperature

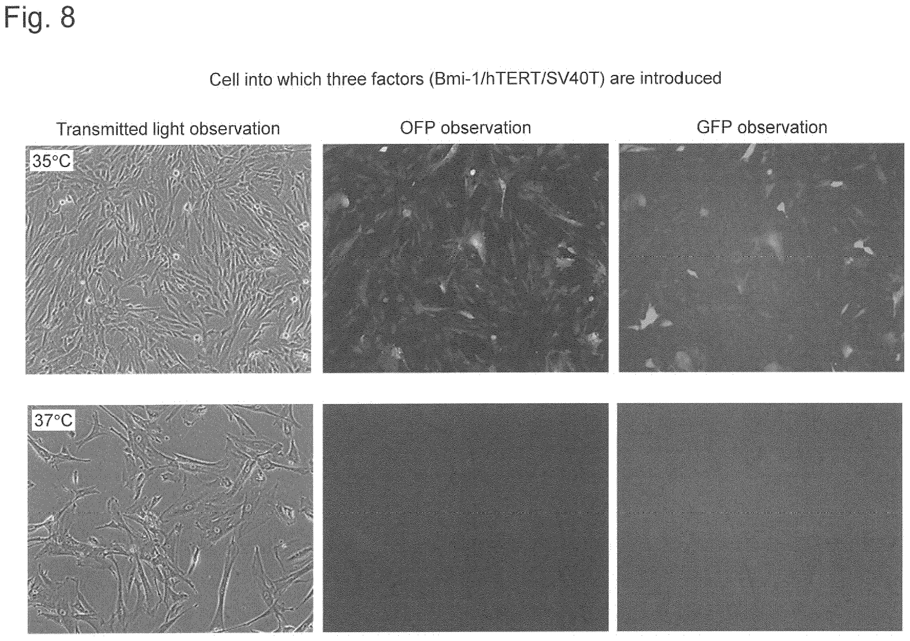

Fluorescent protein expression was confirmed under a fluorescence microscope 10 days after temperature change. As a result, the fluorescence of OFP (co-loaded with Bmi-1) and GFP (co-loaded with hTERT) was confirmed in the cells maintained at the culture temperature of 35°C, whereas the expression was rarely able to be confirmed in the cells at the temperature elevated to 37°C (Figure 8).

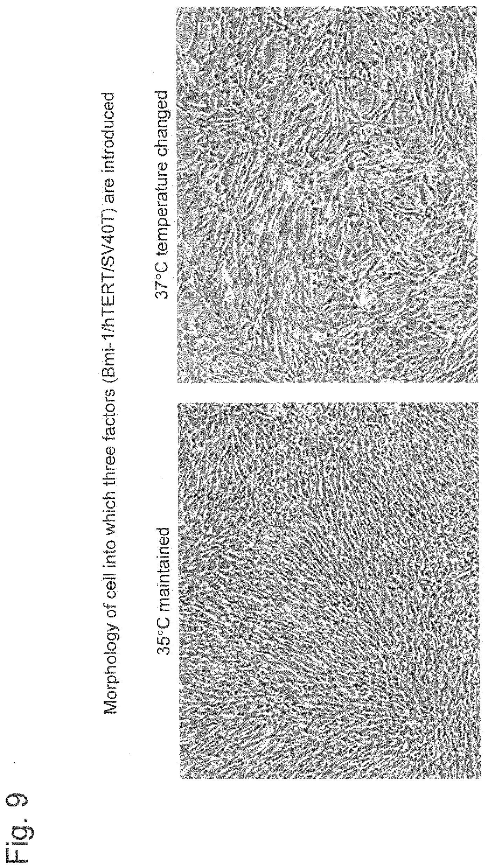

Cell morphology was confirmed 2 weeks after the start of passage culture (92 days after the start of culture). As a result, the cells at 35°C were found to have no change in state and divided actively, whereas the division rate of the cells at 37°C was noticeably decreased so that variations in the sizes of the cells occurred. Thus, a large difference from the homogeneity at 35°C was observed (Figure 9).

From the results described above, the temperature-sensitive effect of the SeV vectors, which was already revealed in iPS cells, was also confirmed in MSCs by temperature change from 35°C to 37°C. The results also suggested that the removal of three immortalizing factors reset cell immortalization and restored finitely proliferative cells.

### (4) Chromosome analysis

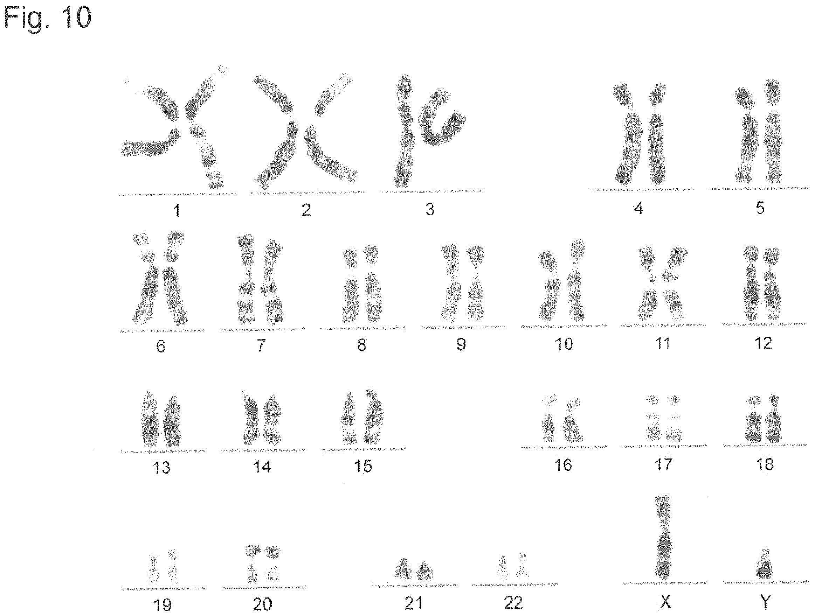

The chromosome analysis of the cells was conducted at the point indicated by downward pointing triangle in Figure 7 by a quinacrine-Hoechst differential staining technique. In the quinacrine-Hoechst differential staining technique, a chromosome slide was first dipped in 50 ml of a Mcilvaine solution (prepared by mixing and autoclaving 280 ml of a 0.1 M citric acid solution and 220 ml of a 0.2 M disodium hydrogen phosphate solution) and subsequently dipped for 30 minutes in Hoechst 33258 (cat: B-2883-25MG, Sigma) dissolved at 10 ng/ml in 50 ml of a Mcilvaine solution. The chromosome slide was washed by running tap water with a weak water stream from the undersurface of the chromosome slide, then dipped again in a Mcilvaine solution for 5 minutes, and then covered and mounted with cover glass using a Mcilvaine mountant (1: 1 mixture of Mcilvaine and glycerol). As a result of conducting analysis using a chromosome analysis microscope (model: AxioImager Z2, ZEISS) and chromosome analysis software (model: Ikaros V5.7.4 CM/V5.4.12, Metasystems), the immortalized cell line had a normal karyotype, as in the parent line, at all the points (Figure 10).

### (5) Evaluation of telomere length

Telomere lengths were measured at the points indicated by * in Figures 6 and 7. The telomere length evaluation was performed by real-time PCR with genomic DNA as a template and the relative quantification of telomere sequences. Genomic DNA extraction from the cells was carried out using Gentra(R) Puregene(R) Kit (Qiagen N.V.) with reference to the procedures of the manufacturer. In the real-time PCR, the primers used were Telomere primer set and Single copy reference primer set attached to Relative Human Telomere Length Quantification qPCR Assay Kit (ScienCell Research Laboratories, Inc.), and the PCR reagent used was FastStart Essential DNA Green Master (Roche). The PCR reaction conditions abided by the conditions recommended by the manufacturer. StepOnePlus (Life Technologies Japan Ltd.) was used in PCR reaction and data acquirement, and the data was analyzed with StepOne Software v2.3.

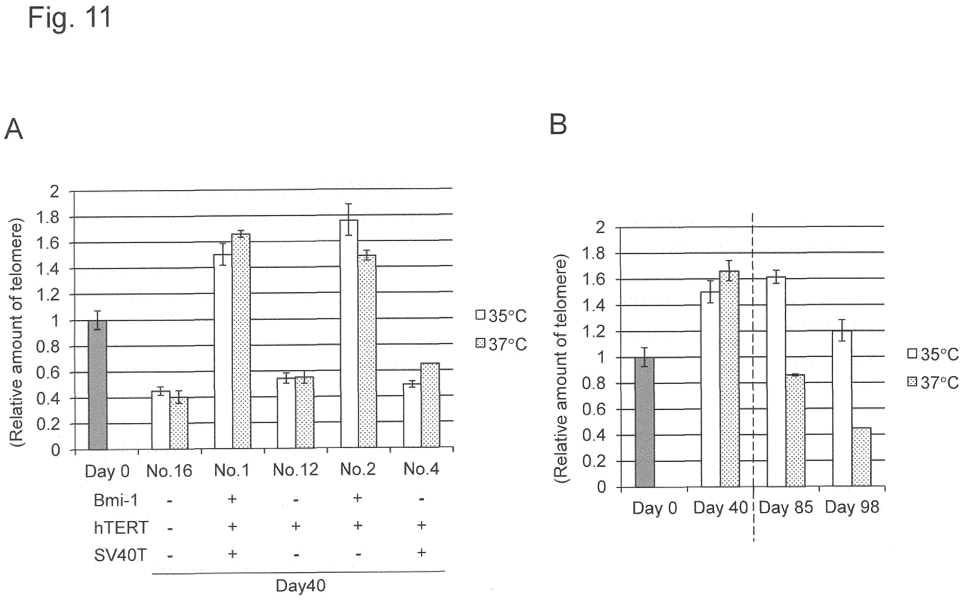

The results are shown in Figure 11. Telomere elongation was confirmed in the combination of three factors Bmi-1, hTERT, and SV40T and the combination of two factors Bmi-1 and hTERT, whereas no elongation was seen in hTERT alone (Figure 11A). Marked shortening of telomere was see in long-term culture after immortalizing factor removal (Figure 11B). Although such shortening was also seen when the immortalizing factors were not removed (35°C), the telomere length was at the same level as in the early hMSCs even in final sampling. The results described above suggested that hTERT is necessary, but is not a sufficient condition, for immortalization in comparison to proliferative potential.

### (Example 4) Differentiation potential of cells of immortalized population

The immortalized cells (MSCs) prepared in Example 3, into which three factors (Bmi-1/hTERT/SV40T) are introduced were examined for their differentiation potential of adipocyte, osteoblast, neuron, or chondrocyte differentiation. Umbilical cord blood-derived MSCs (ATCC PCS-500-010) immortalized with three factors Bmi-1, hTERT, and SV40T and cultured over a long period (cultured for 4 months from gene introduction) were used in the differentiation test. This period of 4 months makes general unimmortalized MSCs difficult to culture.

A commercially available kit for differentiation (PromoCell GmbH) was used in the differentiation of the MSCs. Mesenchymal Stem Cell Adipogenic Differentiation Medium 2 (product code: C-28016) was used for adipocyte differentiation; Mesenchymal Stem Cell Osteogenic Differentiation Medium (product code: C-28013) was used for osteoblast differentiation; Mesenchymal Stem Cell Neurogenic Differentiation Medium (product code: C-28015) was used for neuron differentiation; and Mesenchymal Stem Cell Chondrogenic Differentiation Medium (product code: C-28012) was used for chondrocyte differentiation. Operation methods were carried out in accordance with the attached protocols.

The adipocyte differentiation was confirmed using fluorescent dye Lipi-Green (Dojindo Laboratories: product code: LD02) which specifically stains fat droplets serving as an index for the adipocyte differentiation. The osteoblast differentiation was confirmed by alkaline phosphatase staining. The neuron differentiation was confirmed using fluorescent dye NeuroFluor NeuO (Veritas Technologies Corp.: product code: ST-01801) which specifically stains neurons. The chondrocyte differentiation was confirmed by Alcian blue staining.

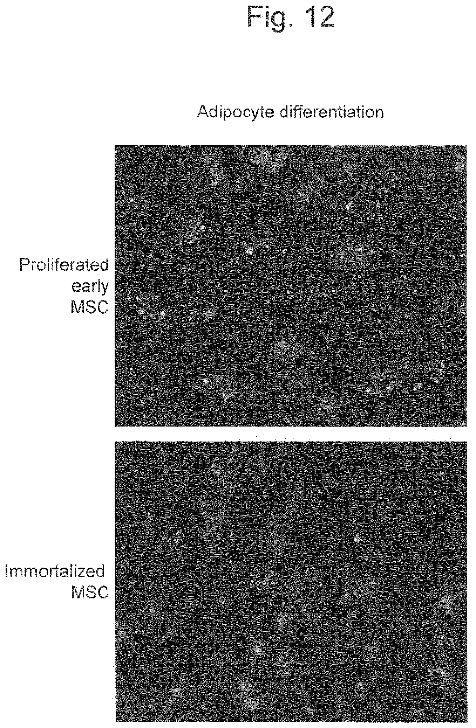

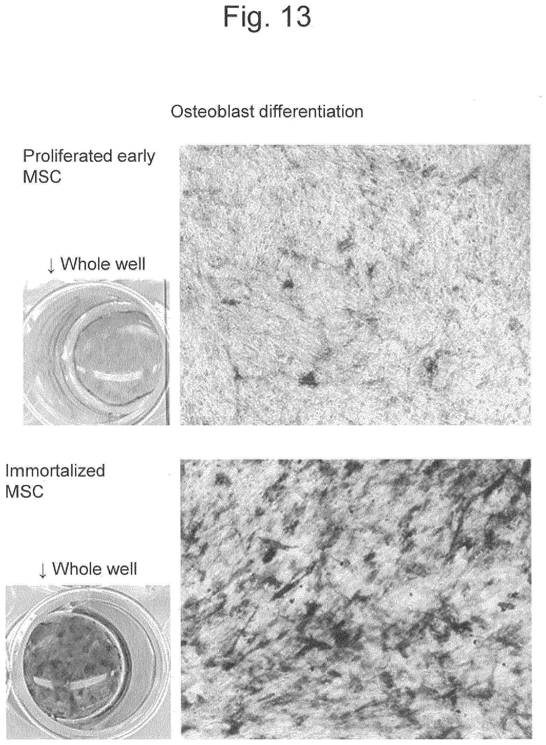

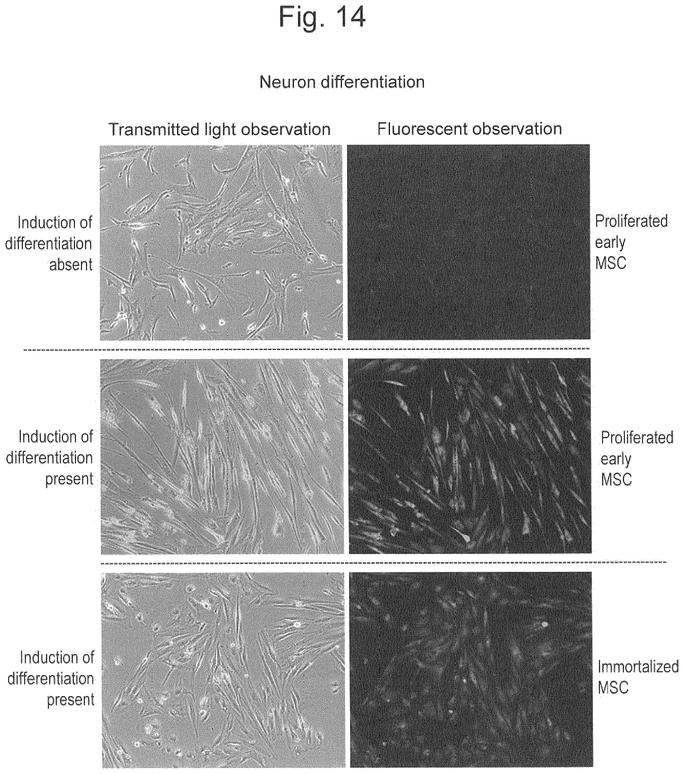

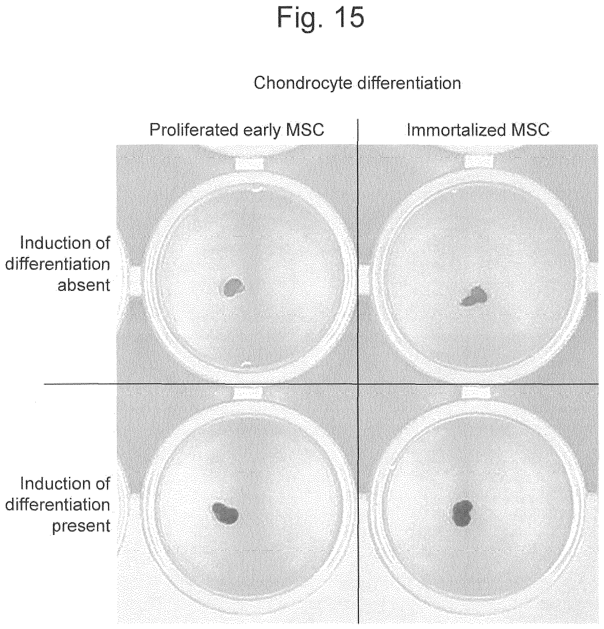

Non-transgenic MSCs that retained differentiation potential in early cells cultured for approximately 2 weeks from purchase, and were at the logarithmic phase were used as a positive control to compare their differentiation potential with that of the immortalized MSCs after a lapse of 4 months. As a result, for adipocyte differentiation, the fat droplets serving as an index for differentiation were observed in both the cells. The immortalized MSCs had a lower degree of differentiation than that of the positive control (Figure 12). For osteoblast differentiation, the alkaline phosphatase stain was confirmed in both the immortalized MSCs and the positive control, which thus differentiated into osteoblasts (Figure 13). The immortalized MSCs had a higher rate of differentiation than that of the positive control. For neuron differentiation, the fluorescence was observed in almost all the cells in both the immortalized MSCs and the positive control, which were thus found to differentiate into neurons (Figure 14). For chondrocyte differentiation, the degree of differentiation was not able to be compared between the immortalized MSCs and the positive control. However, the MSCs induced to differentiate were evidently stained dark with Alcian blue and thus confirmed to differentiate into chondrocytes, as compared with a negative control (induction of differentiation absent) (Figure 15). From the results described above, it was confirmed for the immortalized cells (MSCs) into which three factors (Bmi-1/hTERT/SV40T) are introduced that even the MSCs after a lapse of 4 months maintained multilineage potential, as in the positive control MSCs.

### (Example 5) Cloning test of immortalized MSC

The immortalized cells (MSCs) prepared in Example 3, into which three factors (Bmi-1/TERT/SV40T) are introduced were subjected to a confirmatory experiment on whether these cells could be cloned or how difficult it was to clone unimmortalized MSCs.

Cloning was performed using three types of MSCs: non-transgenic umbilical cord blood-derived MSCs (ATCC PCS-500-010) that retained differentiation potential in early cells cultured for approximately 2 weeks from purchase, and were at the logarithmic phase; immortalized MSCs immediately after gene introduction (after a lapse of 2 weeks); and immortalized MSCs cultured for a long period from gene introduction (after a lapse of 4 months).

These three types of MSCs maintained by culture were completely dissociated into single cells once, and the numbers of cells were measured. A 96-well plate divided into four zones each involving 24 wells was inoculated with varying numbers of cells such that the zones included 4 cells, 8 cells, 16 cells, and 32 cells/100 µl, respectively, per well. The cells were cultured at 35°C in a 5% CO₂ incubator until the single cells formed colonies. Medium replacement was performed by replacing half the amount every 3 days so as not to disturb colony formation.

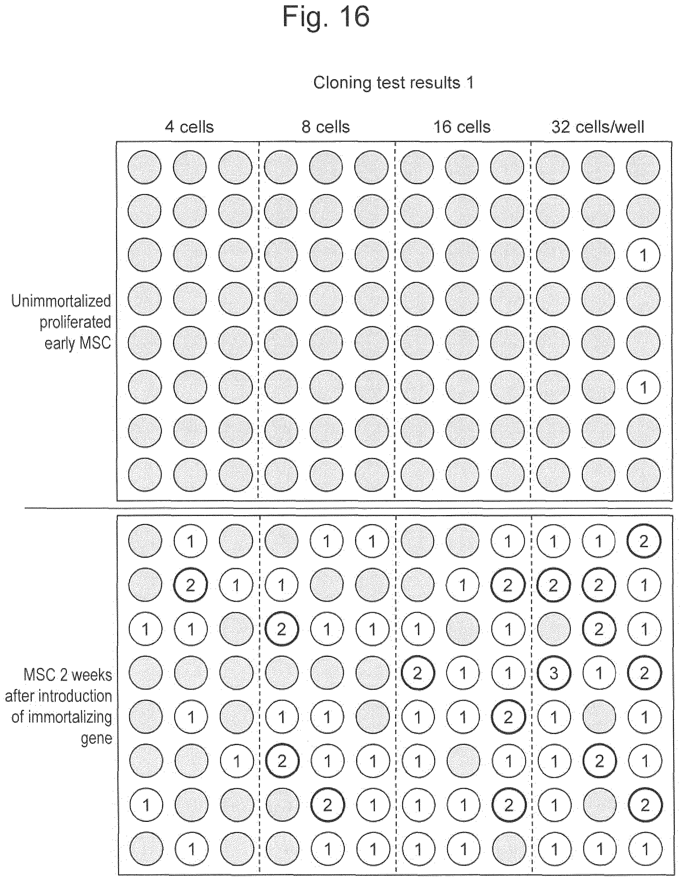

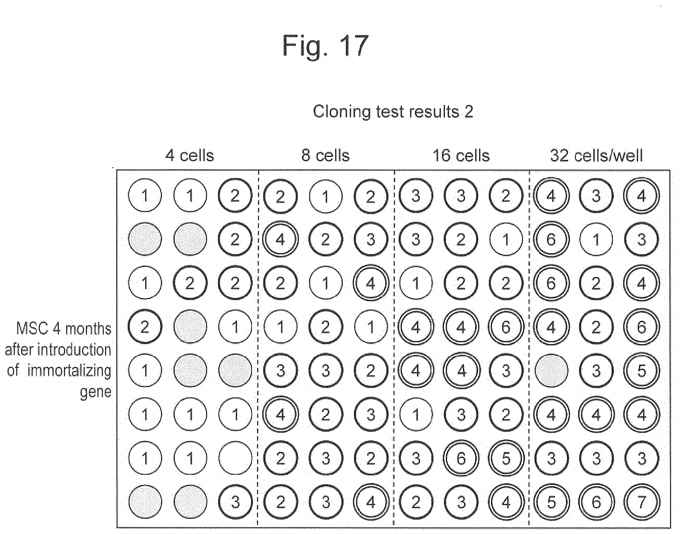

When the cells cultured for 10 days were observed under a microscope, the single cells proliferated into colonies having a sufficient size. Accordingly, the number of colonies formed per well was measured, and the pattern thereof was schematically summarized in drawings (Figures 16 and 17). The numeral in each of the 96 wells in each drawing represents the number of formed colonies.

The ease of colony formation can be confirmed at a glance from the results. The unimmortalized proliferated early MSCs, which correspond to general MSCs, formed colonies in only 2 wells in the zone of 32 cells/well. On the other hand, the MSCs into which the immortalizing genes are introduced using the SeV vectors formed colonies with a high frequency of 9 wells/24 wells, even at a low density of 4 cells/well, 2 weeks later which was not long since introduction (Figure 16). After a lapse of 4 months, these MSCs formed colonies in more than half the number of wells (16 wells/24 wells) even at a low density of 4 cells/well (Figure 17).

The results described above revealed that even MSCs which are difficult to clone can be easily cloned by introduction of three immortalizing genes using the SeV vector, irrespective of a subsequent elapsed time. Cloning feasible at any time is very useful from the viewpoint of the quality control of regenerative medicine.

### (Example 6) Various analyses of cloned immortalized MSC

### (1) Proliferative potential of cloned cell

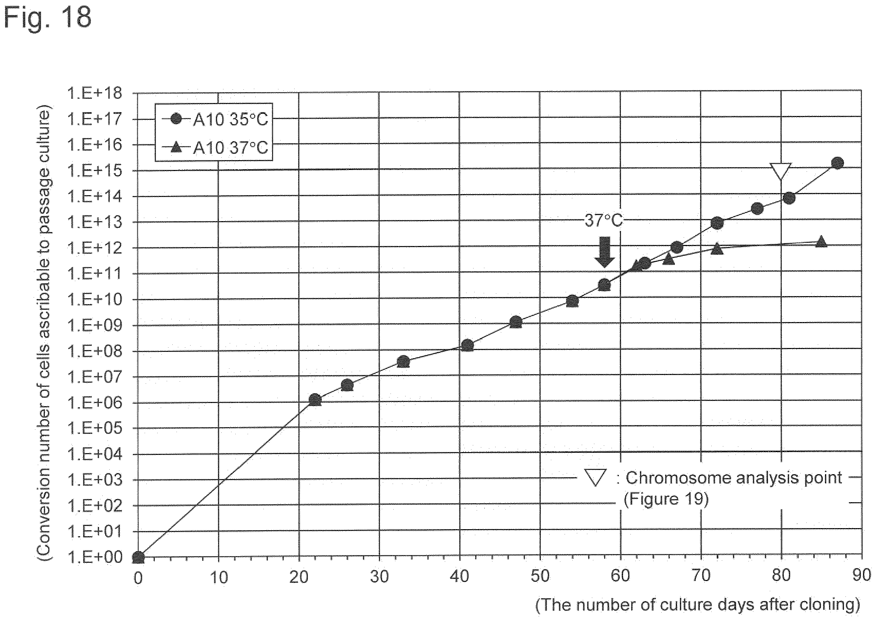

Five clones of MSCs obtained by dissociation into single cells and cloning 2 weeks after SeV vector infection were cultured for 60 days and then cultured at 35°C or 37°C. The proliferation of 3 of the 5 clones was reduced by the removal of the SeV vectors at 37°C. The proliferation curve of clone A10 among these clones is shown (Figure 18).

### (2) Confirmation of SeV vector removal by temperature change

The presence of the SeV vectors was able to be confirmed in the group of 35°C, as in the population culture experiment, whereas the presence of the SeV vectors was not able to be confirmed in the group of 37°C.

### (3) Chromosome analysis of cloned cell

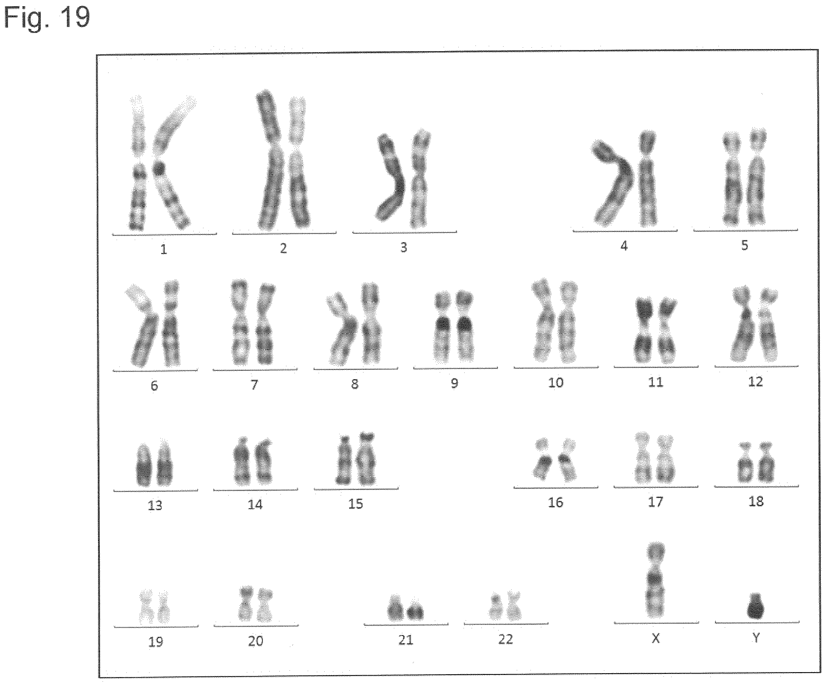

As a result of conducting chromosome analysis on 10 clones obtained by cloning, 8 clones had a normal karyotype. The karyotype analysis results about clone A10 whose cell proliferation is shown in Figure 18 among these clones are shown (Figure 19).

### (4) Differentiation potential of cloned immortalized MSC

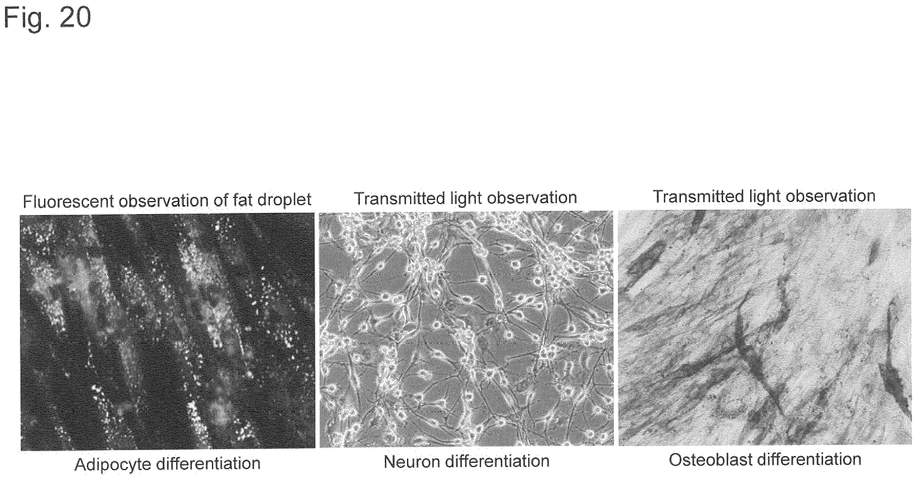

Among the cloned immortalized MSCs, 5 clones were examined for their adipocyte, osteoblast, or neuron differentiation potential by the same method as in Example 4. As a result, the degree of differentiation was found to differ, as in the morphological difference found depending on clones. However, all the clones were found to differentiate into adipocytes, osteoblasts, or neurons and maintained multilineage potential (Figure 20). Some clones had a much better state of differentiation than that of the cells before cloning, and not only neural marker expression but a formed neural network in which neurites spread in a netlike pattern was observed in neuron differentiation.

### (Example 7) Immortalization of MSCs differing in cell age (degree of senescence) and single-cell cloning

A cell population obtained by proliferation from one MSC has the same genetic properties and therefore has constant quality and facilitates quality control. In this respect, single-cell cloning has a significant meaning in regenerative medicine. Furthermore, in the case of gene introduction from the outside, single-cell cloning is also indispensable for selecting only the cells of interest.

MSCs isolated from the human body are a cell mixture of various stages from young cells to senescent cells. The proportions thereof also differ among individuals, and the proliferation of only cells isolated at a particular stage (youth) is difficult. To put it the other way around, if MSCs of any stage from young cells to senescent cells can be immortalized and can be further cloned, the range of application thereof to regenerative medicine is drastically expanded.

Accordingly, three types of cells, young cells, senescent cells, and cells of an intermediate stage therebetween, were immortalized and examined for whether single-cell cloning could be performed.

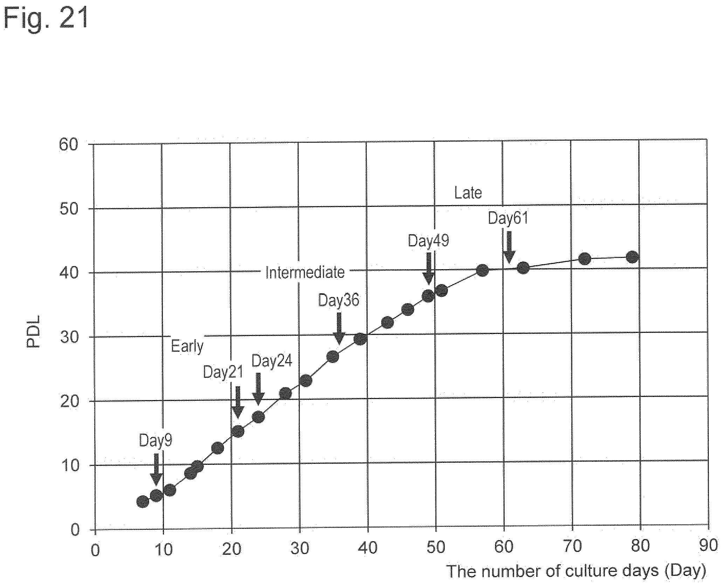

The cells used were umbilical cord blood-derived MSCs (product name: Umbilical Cord-Derived Mesenchymal Stem Cells; Normal, Human (ATCC PCS-500-010)). The purchased cells were cultured at 37°C for a long period of approximately 80 days until proliferation was arrested. In the meantime, some of the cells were cryopreserved at approximately 1-week intervals of passages (Figure 21).

Among the cryopreserved cells, three types of cells, early, intermediate, and late cells (Days 9, 24, and 49), were thawed at the same time, cultured for 11 days in order to suppress the influence of freezing-thawing, and inoculated to 24-well plates (24 wells each, 1 × 10⁵ cells/well). On the next day, the cells were immortalized by infection with the SeV vectors loaded with three factor (Bmi-1/hTERT/SV40T) genes at MOI: 20 (Days 21, 36, and 61).

After induction of immortalization, the cells were transferred to a CO₂ incubator of 35°C from 37°C and expansion-cultured for 8 days (24 wells -> 6 wells). Then, the immortalization-induced cells were inoculated to 96-well plates such that the number of cells per well was 1, 2, or 4. Control cells without the induction of immortalization were inoculated such that the number of cells per well was 5, 10, 15, or 20. The cells were cultured for approximately 2 weeks until a cell population (colony) appeared by the division and proliferation of one cell. The number of generated colonies was counted by microscopic observation.

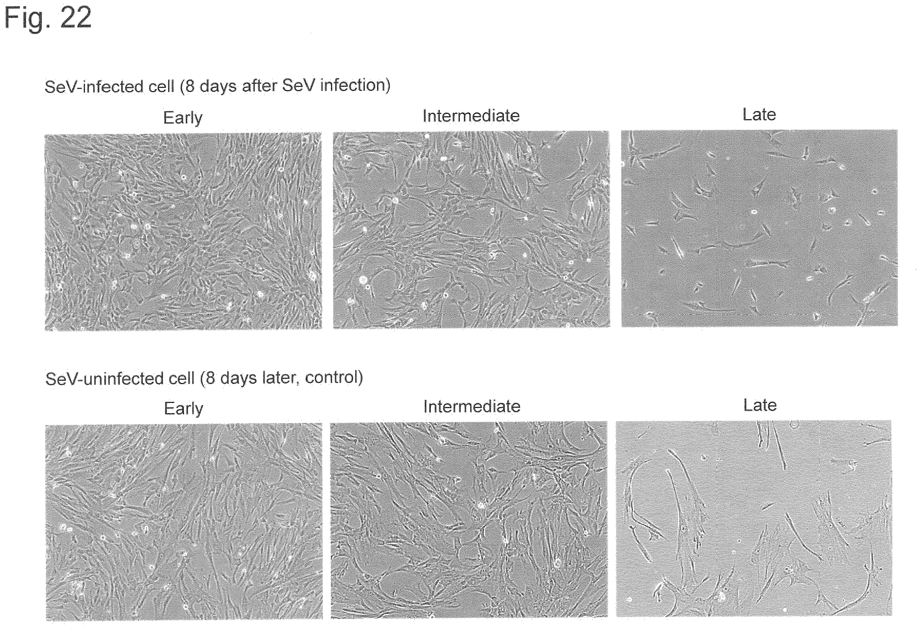

When the SeV vector-infected cells and the SeV vector-non-infected cells of three types of MSCs differing in cell age (early, intermediate, and late) were morphologically observed immediately before inoculation to 96-well plates (8 days after SeV vector infection), a marked difference in appearance also occurred therebetween (Figure 22).

As shown in Figure 22, a cell density which reflected the number of cells was higher in younger cells, revealing that cell proliferation was more active. Although a marked difference in the number of cells depending on the presence or absence of SeV vector infection did not appear, evident increase in the number of cells was observed in the SeV vector-infected cells of the late cells. Particularly, cell morphology or size varied markedly. As the time passed, the SeV vector-non-infected control cells became giant and their division rate was decreased. By contrast, the SeV vector-infected cells divided actively and had the same level of size, and the early, intermediate, and late cells were no longer distinguishable by size.

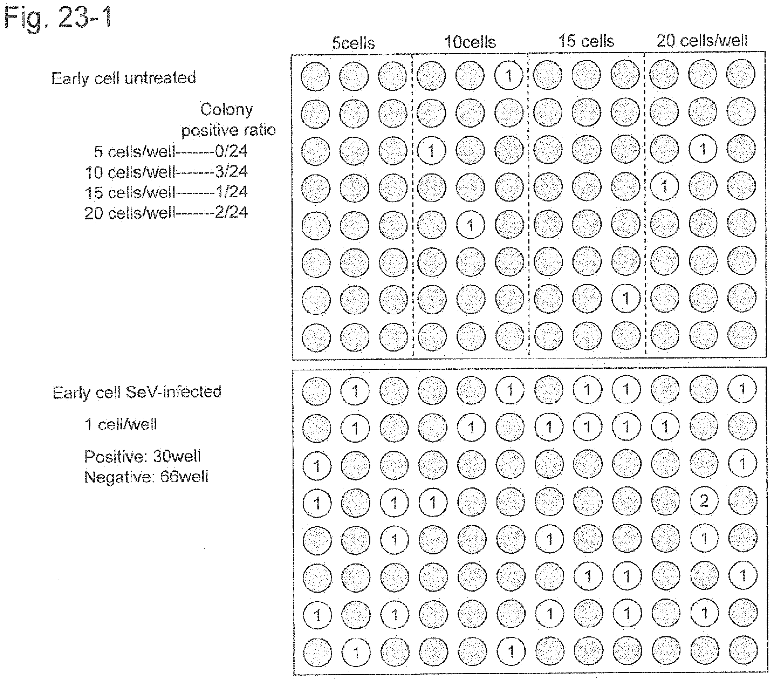

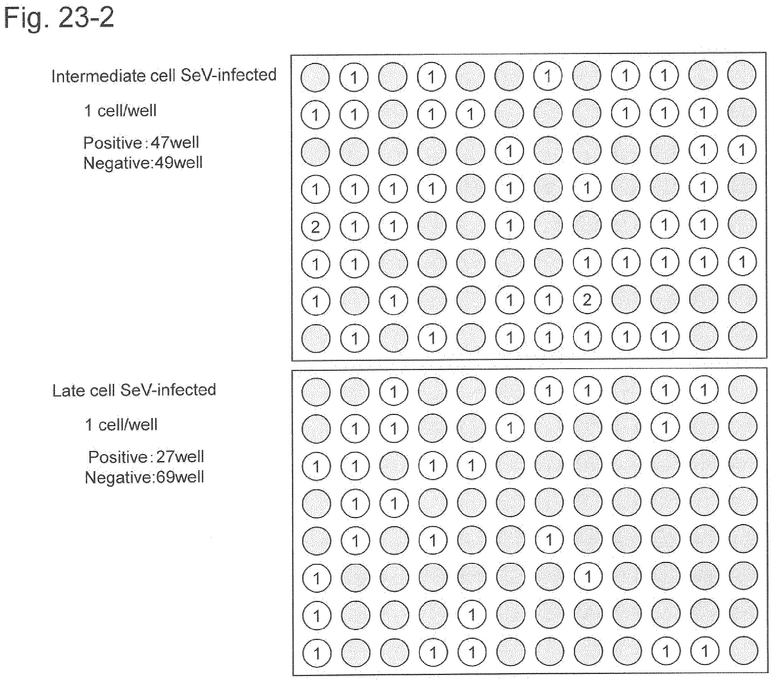

As a result of cloning in 96 wells, no colony appeared from 5 cells per well of the SeV vector-non-infected cells, confirming that cloning was difficult, as in Example 5. In the SeV vector-infected cells, many colonies were observed in the single-cell cloning plates inoculated at a proportion of one cell per well (Figures 23-1 and 23-2).

Colonies were observed in 30 out of 96 wells for the early cells, observed in 47 wells for the intermediate cells, and observed in 27 wells for the late cells. These results demonstrated that MSCs immortalized by induction with the SeV vectors are capable of easily obtaining a cell population derived from one cell, irrespective of the degree of senescence thereof.

### (Example 8) Confirmation of effect on MSC that arrested its proliferation

The effect of the SeV vectors loaded with the immortalizing genes was confirmed on MSCs that completely arrested their cell division due to senescence.

Cryopreserved cells in a state where cell proliferation was almost arrested (Day 72) were thawed and inoculated to a 24-well plate. Cells confirmed to not proliferate while the influence of freezing-thawing was suppressed (Day 90) were infected with the SeV vectors (24 wells, 1 × 10⁵ cells/well, MOI: 20). Then, change in cells was observed under a microscope.

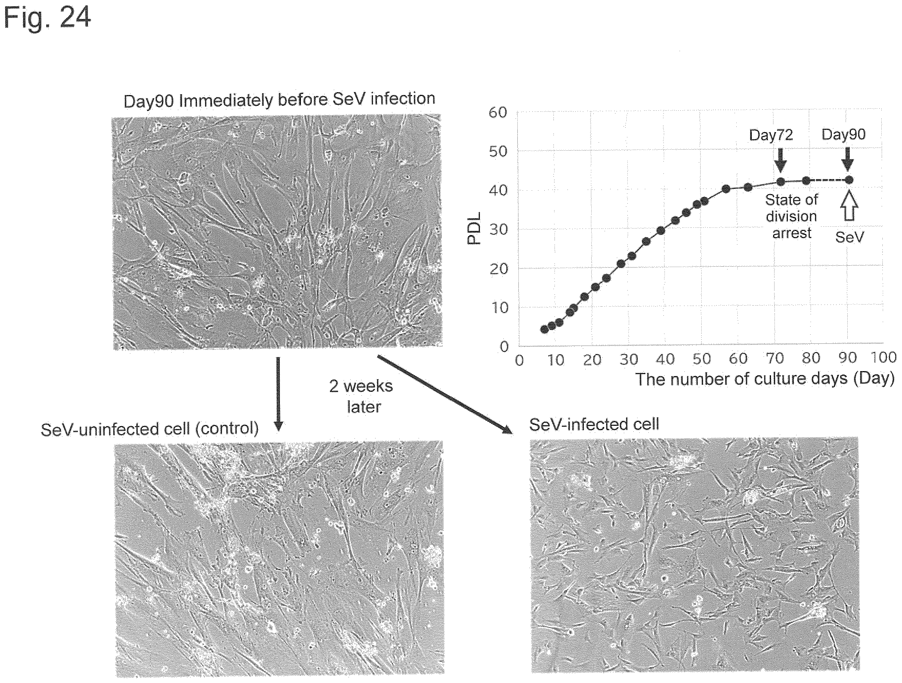

SeV vector-non-infected cells (control cells) had no large change, and the whole cells were observed to be rounded and dead. On the other hand, in the SeV vector-infected cells, although no change was seen for several days, many cells were rounded 1 week later and seemed to be killed as they were. Nonetheless, low adherent cells were observed among the cells and converted to a cell population that proliferated as actively as immortalized cells 2 weeks later (Figure 24).

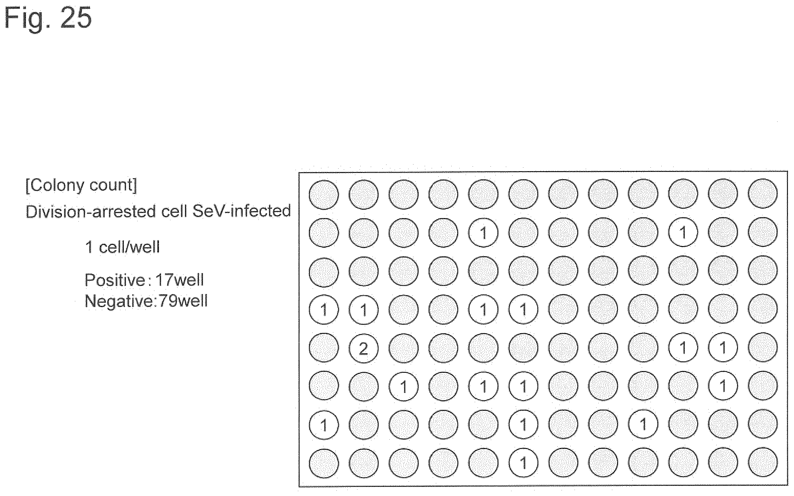

The cells started re-proliferation were subjected to single-cell cloning in the same manner as in Example 7. As a result, a cell population that proliferated was confirmed in 17 out of 96 wells, though this proportion was lower than the aforementioned results about the single cells in Figures 23-1 and 23-2. Thus, even the cells that completely arrested their division were confirmed to start division again by infection with the SeV vectors and to permit single-cell cloning (Figure 25).

Unlike cell proliferation associated with cell immortalization, this change was considered to remove the cytoplasm unnecessary for cell proliferation. Although a detailed mechanism is unknown, the cells that completely arrested their proliferation due to senescence re-proliferated. In this case, the phrase "cell rejuvenation" rather than "immortalization" holds true.

### (Example 9) Study on cell proliferation of human mesenchymal stem cell in serum-free medium

Fat tissue-derived human mesenchymal stem cells hMSC-AT (PromoCell GmbH; C-12977) and bone marrow-derived human mesenchymal stem cells hMSC-BM (PromoCell GmbH; C-12974) were used in an experiment. When the day when hMSC-AT and hMSC-BM were infected with the SeV vectors was defined as the "date of infection", hMSC-AT-non-infected cells and -infected cells were cultured in a serum-containing medium up to 13 days and 11 days, respectively, from the date of infection, and hMSC-BM-non-infected cells and - infected cells were cultured in a serum-containing medium up to 11 days and 17 days, respectively, from the date of infection. The serum-containing medium used was a medium having the composition of D-MEM (low glucose), 20% FBS, 0.01 mol/L Hepes, 100 units/ml penicillin, 100 µg/ml streptomycin, and 20 ng/ml bFGF. Then, the medium was replaced with a serum-free medium, and the culture was continued. The serum-free medium used was Stem Fit For Mesenchymal Stem Cell (Ajinomoto Co., Inc.; A3) medium. The culture dish used was a 6 cm dish (Corning Inc.; 353004) coated with iMatrix-511 silk (Matrixome Inc.; 892091).

The SeV vector infection was performed under conditions involving 2 × 10⁵ cells of the hMSC-AT cells or the hMSC-BM cells and MOI: 40 of the SeV vectors. The non-infection condition and the infection condition were each carried out once. The medium was replaced with a fresh one 24 hours after infection, and maintenance culture was continued. The cell culture was performed at 35°C in a 5% CO₂ incubator.

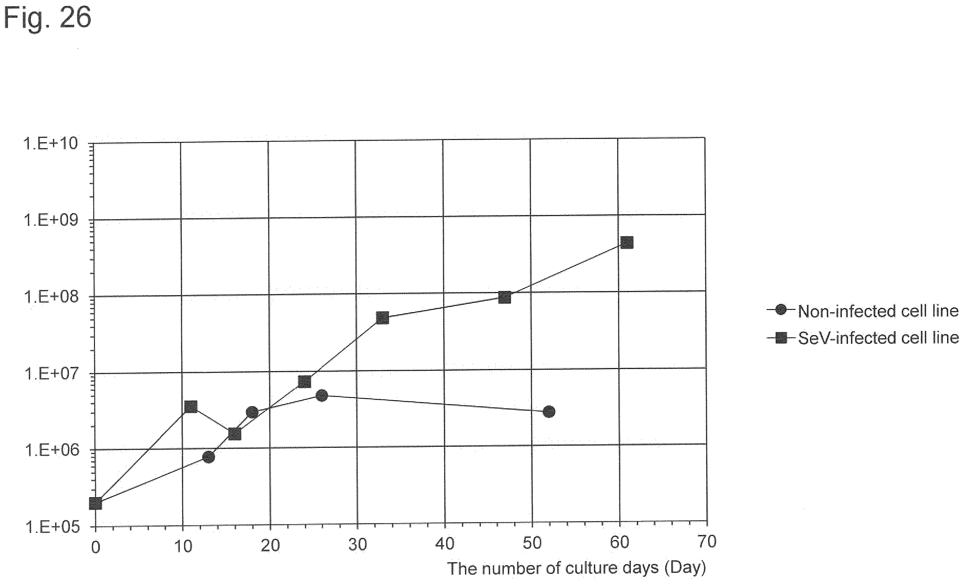

In the experiment of the hMSC-AT cells, the culture of the non-infected cell line was terminated on 52 days from the date of infection because decrease in the number of cells was found. On the other hand, the SeV vector-infected cell line continued to proliferate even on day 52 or later (Figure 26).

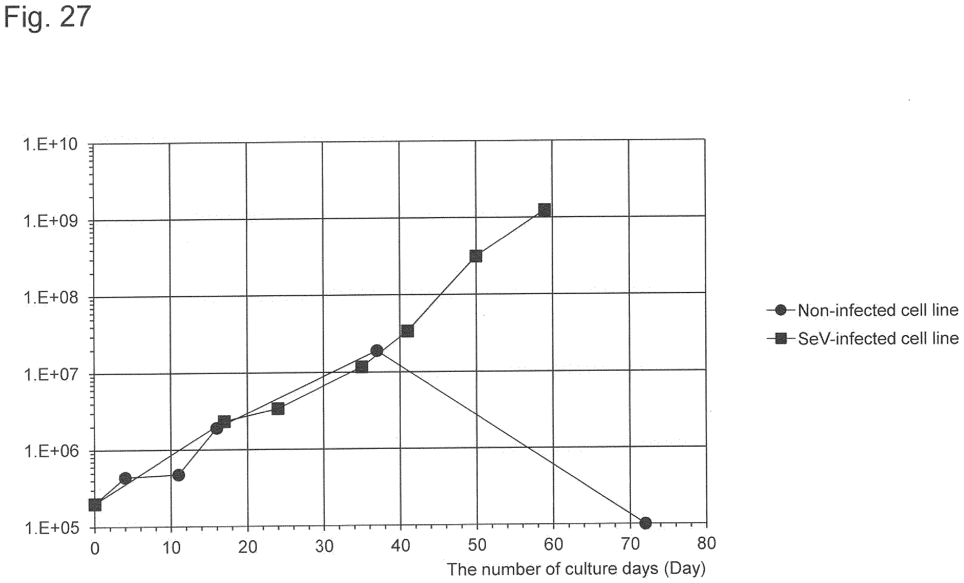

In the experiment of the hMSC-BM cells as well, the non-infected cell line arrested its cell proliferation and no longer proliferated on 40 days or later from the date of infection, and the number of cells was measured on day 72. As a result, the number of cells was equal to or lower than the detection limit. Therefore, the cell culture was terminated at this point in time. On the other hand, the SeV vector-infected cell line continued to proliferate even on day 40 (Figure 27).

From the results described above, it was confirmed that: SeV vector infection can also extend cell division potential for fat tissue-derived and bone marrow-derived MSC cells, as in the umbilical cord blood-derived MSC cells; and even infected cells can continue to proliferate in a serum-free medium, as in the serum-containing medium.

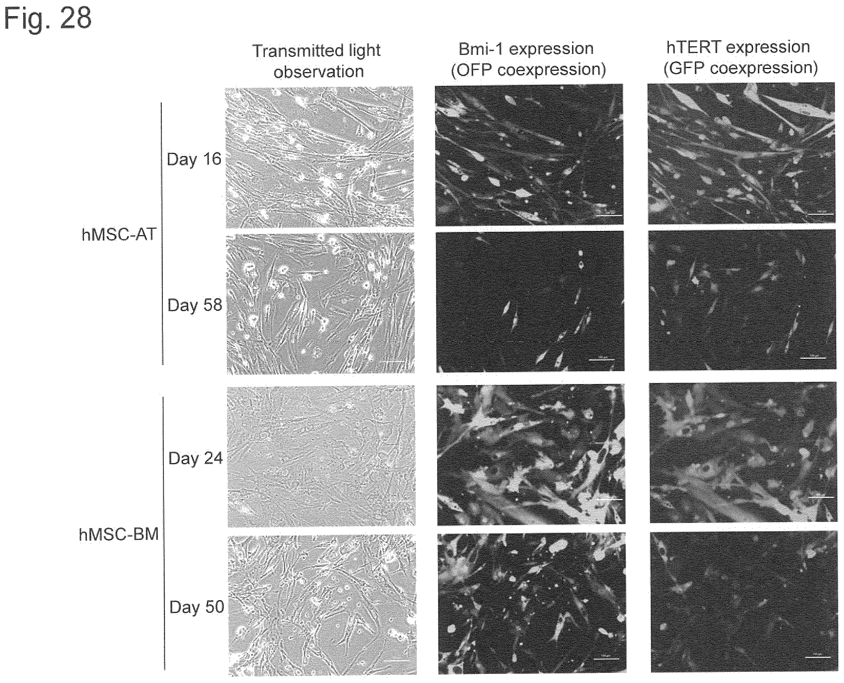

After SeV vector infection, the fluorescent observation of GFP (hTERT) and OFP (Bmi-1) was performed. As a result of comparing the early stage of culture of the hMSC-AT cells and the hMSC-BM cells in the serum-free medium (16 and 24 days from the date of infection) with the stage after the culture in the serum-free medium was continued (58 and 50 days from the date of infection), the expression of GFP and OFP was found to be maintained (Figure 28). From these results, it was confirmed that SeV can be maintained even if culture is continued in a serum-free medium, as in the culture in the serum-containing medium.

### (Example 10) Study on cell proliferation of immortalized rat MSC

Rat subcutaneous fat-derived mesenchymal stem cells rMSC (Cosmo Bio Co., Ltd., MSA01C) were cultured in a medium for rat subcutaneous fat-derived mesenchymal stem cell proliferation (Cosmo Bio Co., Ltd., MSA-GM).

The SeV vector infection was performed under conditions involving 2 × 10⁵ cells of the rMSC cells and MOI: 40 of the SeV vectors. The non-infection condition was carried out once, and the condition for the infected cell line was carried out twice. The medium was replaced with a fresh one 24 hours after infection, and maintenance culture was continued. The culture dish used was a collagen-coated dish (IWAKI, 4810-010), and the cells were maintained at 35°C in a 5% CO₂ incubator.

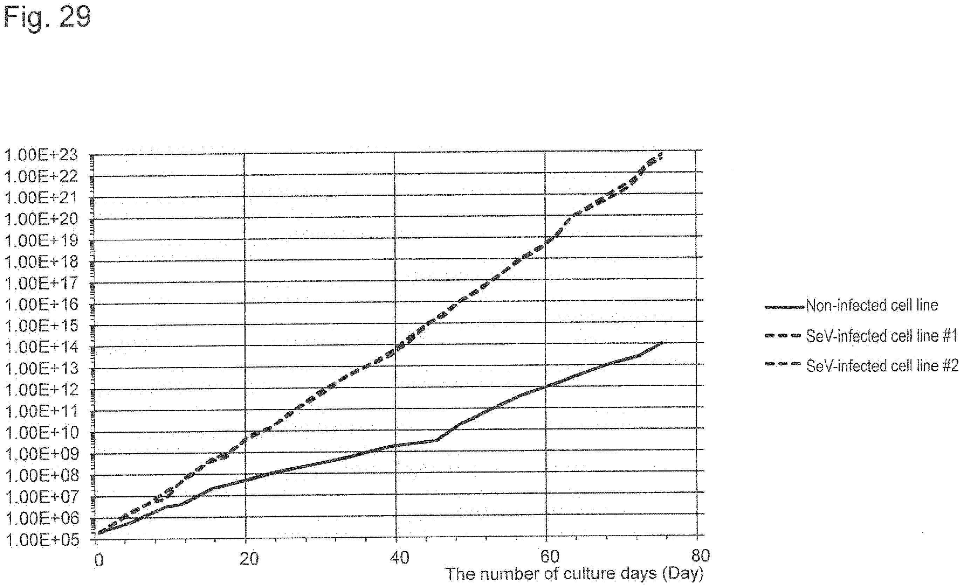

As a result of carrying out long-term culture, a phenomenon was seen in which the non-infected cells of the rMSC cell line, the proliferative potential of which is originally reduced by several passages, continued to proliferate (Figure 29). This is presumably because the rMSC cell line was accidentally immortalized. However, as a result of performing culture for 75 days, the infected cells were found to have a proliferation rate two or more times faster than that of the non-infected cells.

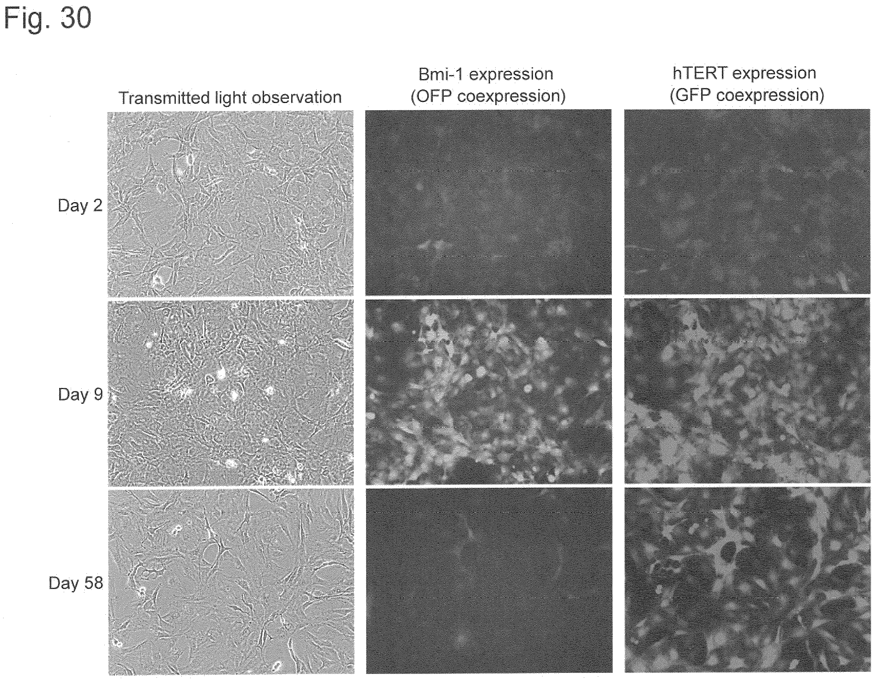

After SeV vector infection, the fluorescent observation of GFP (hTERT) and OFP (Bmi-1) was performed. Fluorescent photographs were taken under an all-in-one microscope (Keyence Corp.; BZ-X800). The photographing was performed under conditions involving a 20× magnification and exposure times of 1/5 seconds and 1/20 seconds for GFP and OFP, respectively. As a result of performing fluorescent observation, the expression of OFP (Bmi-1) and GFP (hTERT) was found under the condition of 35°C even after culture for 58 days after infection (Figure 30).

### (Example 11) Study on cell proliferation of immortalized HFL1

Human fibroblasts (HFL1 cells, RIKEN: RCB0521) were cultured in Ham-F12 medium (Nacalai Tesque, Inc.; 17458-65) supplemented with 15% (v/v) of inactivated fetal bovine serum (Nichirei Corp.; 175012) and 100 units/mL of a penicillin-streptomycin solution (FUJIFILM Wako Pure Chemical Corp.; 168-23191).

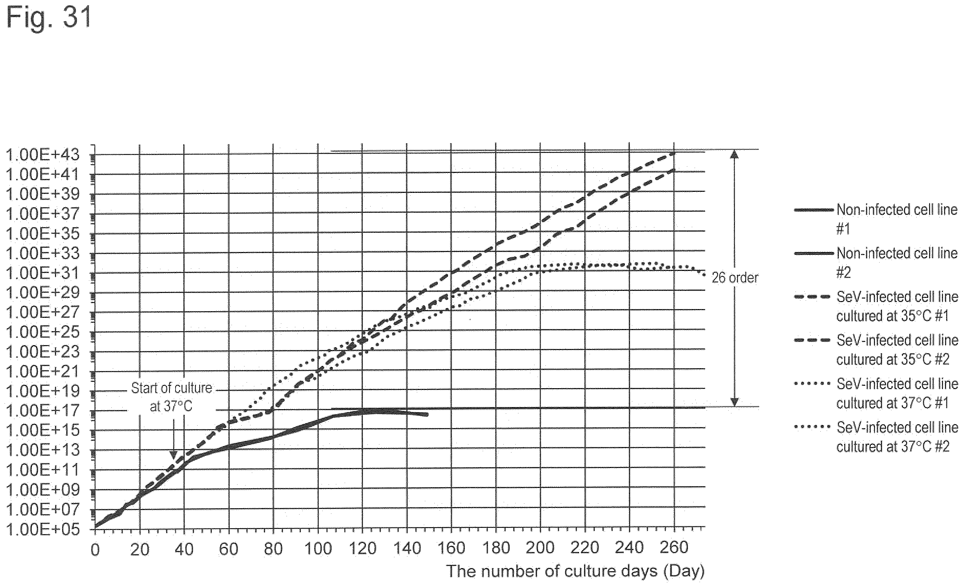

The SeV vector infection was performed under conditions involving 2 × 10⁵ cells of the HFL1 cells and MOI: 40 of the SeV vectors. The non-infection condition and the infection condition were each carried out twice. The medium was replaced with a fresh one 24 hours after infection, and maintenance culture was continued. The infected cells were divided 34 days after infection to two conditions: culture at 35°C and culture at 37°C (Figure 31, arrow). The cell culture was performed at each temperature in a 5% CO₂ incubator.

The culture of the non-infected cells was terminated on 148 days from the date of infection because decrease in the number of cells was found. On the other hand, the culture of the infected cell line was terminated on 259 or 273 days from the date of infection because the cells cultured at 37°C no longer proliferated (Figure 31). This demonstrated that SeV vector infection can also extend cell division potential for HFL1 cells.

The degree of cell proliferation from the start of culture was examined by calculation. As a result, the non-infected cells arrested their proliferation at 17 orders as the number of cells, whereas the SeV vector-infected cells cultured at 37°C arrested their proliferation at 31 orders. The SeV vector-infected cells cultured at 35°C proliferated up to near 43 orders without arresting their proliferation within the culture period, and differed by 26 orders in the number of cells from the SeV vector-non-infected cells in 260 days. From the results described above, it was confirmed that more cells of human fibroblasts HFL1 can proliferate by SeV vector infection.

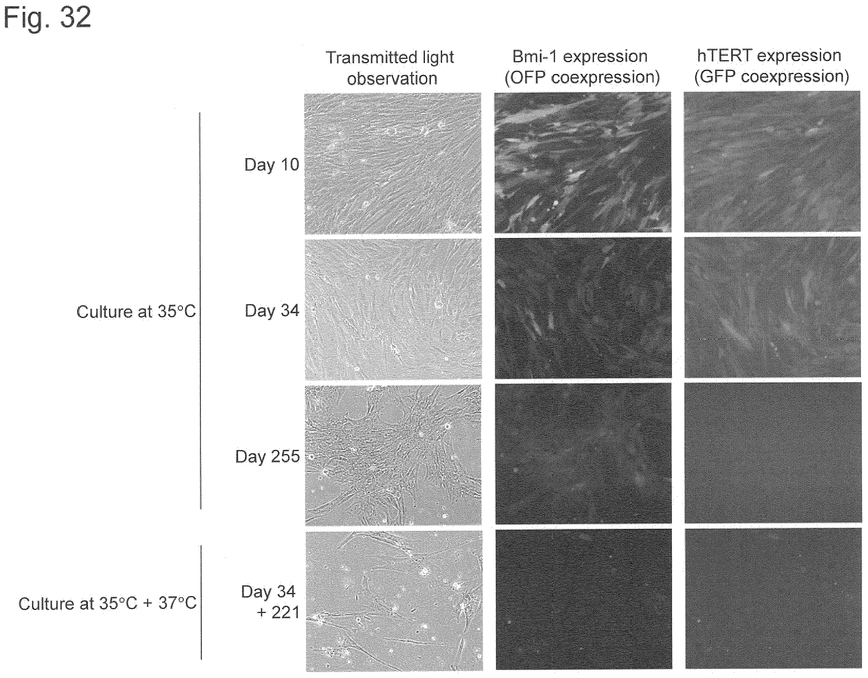

After SeV vector infection, the fluorescent observation of GFP (hTERT) and OFP (Bmi-1) was performed. The results are shown in Figure 32. The fluorescent signals of GFP and OFP were exhibited on 34 days from the date of infection, as in the cells on 10 days from the date of infection. The cells were further cultured for 221 days under respective conditions of 35°C and 37°C. For the cells continuously cultured at 35°C, the expression of GFP was markedly decreased in the cells on day 255 compared with day 34, whereas the expression of OFP was maintained, as in days 10 and 34. On the other hand, for the cells cultured at 37°C, both the GFP- and OFP-positive ratios were markedly decreased as compared with the cells on days 10 and 34. From these results, temperature-sensitive removal of SeV was confirmed.

Cells with advanced cellular senescence may generally exhibit features such as an increased size of the cells, a flat shape, and vacuole formation. The infected cells cultured at 35°C did not exhibit the features of cellular senescence as compared with the infected cells cultured at 37°C. From these results, it was confirmed that cellular senescence in terms of cell morphology was less advanced in the cells continuously cultured at 35°C.

### (Example 12) Study on cell proliferation of immortalized HUVEC

Human umbilical vein endothelial cells (HUVEC cells, PromoCell GmbH, C-12205) were cultured in a medium for endothelial cells (ScienCell Research Laboratories, inc.; 1001).

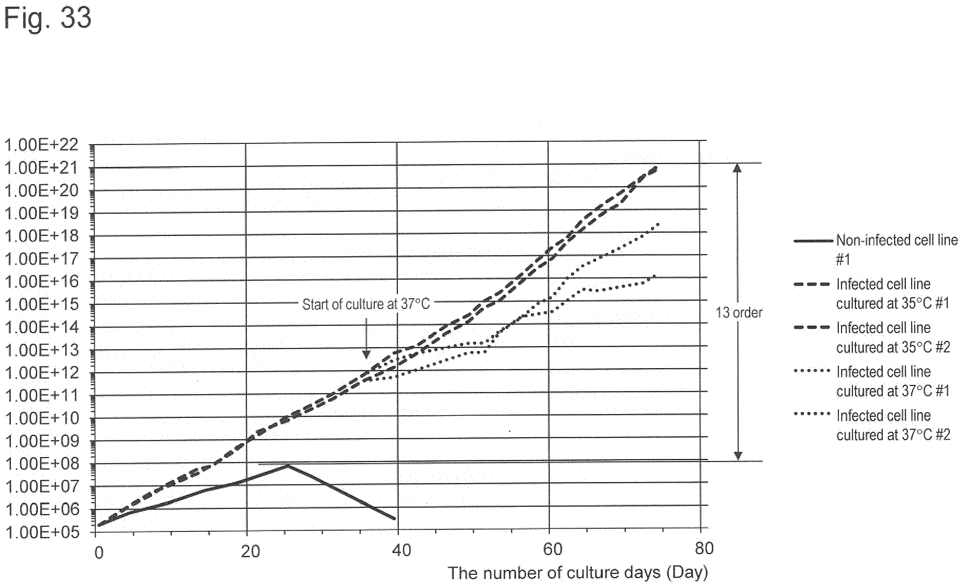

The SeV vector infection was performed under conditions involving 2 × 10⁵ cells of the HUVEC cells and MOI: 40 of the SeV vectors. The non-infection condition was carried out once, and the infection condition was carried out twice. The medium was replaced with a fresh one 24 hours after infection, and maintenance culture was continued. The infected cells were divided 35 days after infection to two conditions: culture at 35°C and culture at 37°C (Figure 33, arrow). The cell culture was performed at each temperature in a 5% CO₂ incubator.

The culture of the non-infected cells was terminated on 39 days from the date of infection because decrease in the number of cells was found. On the other hand, the infected cells continued to proliferate even on 74 days from the date of infection (Figure 33). This demonstrated that SeV vector infection can also extend cell proliferation for HUVEC cells.

The degree of cell proliferation from the start of culture was examined by calculation. As a result, the non-infected cells arrested their proliferation near 8 orders as the number of cells, whereas the SeV vector-infected cells cultured at 37°C proliferated up to 18 orders. The SeV vector-infected cells cultured at 35°C proliferated up to near 21 orders and differed by 13 orders in the number of cells from the SeV vector-non-infected cells in 74 days. From the results described above, it was confirmed that more cells of human umbilical vein endothelial cells (HUVEC cells) can proliferate by SeV vector infection.

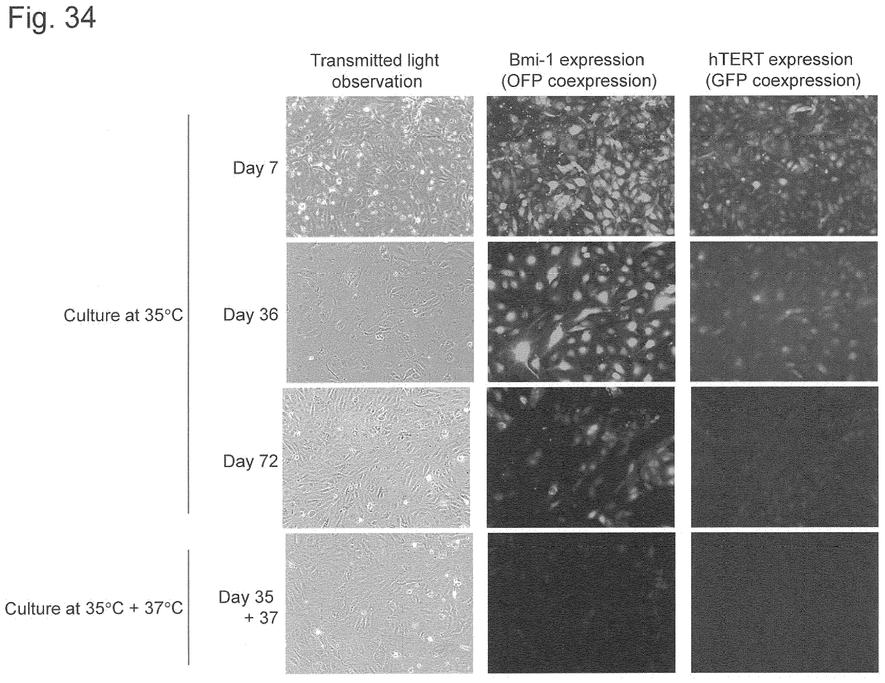

After SeV vector infection, the fluorescent observation of GFP (hTERT) and OFP (Bmi-1) was performed. The results are shown in Figure 34. On 36 days from the date of infection, the cells continuously cultured at 35°C exhibited the fluorescent signals of GFP and OFP similar to those of the cells on 7 days from the date of infection. Then, the cells were further cultured for 37 days under respective conditions of 35°C and 37°C. As a result, for the cells continuously cultured at 35°C, the proportion of fluorescence-positive cells was decreased as compared with the cells on days 7 and 37 from the date of infection, whereas fluorescent signals were found in many cells. On the other hand, for the cells cultured at 37°C, the proportion of fluorescence-positive cells was markedly decreased as compared with the cells on days 7 and 36. From these results, temperature-sensitive removal of SeV was confirmed.

### (Example 13) Stability after introduction of multi-growth factor-inserted human artificial chromosome vector into immortalized MSC cell and long-term culture, and induction of differentiation

### (1) Microcell-mediated chromosome transfer and isolation of drug-resistant clone

The chromosome-donating cells used were CHO cells harboring 21HAC2 loaded with HGF (hgf), GDNF (gdnf), IGF-1 (igf-1), and luciferase (e-luc) described in Watanabe at al. (Mol Ther Nucleic Acids. 2015). The chromosome-accepting cells used were hMSC-UC No. 3 cells which were the immortalized MSC cells described in Example 1. Microcell-mediated chromosome transfer and culture were performed by the method described by Katoh et al. (BMC Biotechnology, 2010, 10: 37). Resistant colonies appeared by culture for 1 week under BS selective culture. A total of 9 colonies obtained by four fusion rounds were isolated and allowed to proliferate, and the following analysis was conducted.

### (2) Confirmation of transferred chromosome

### (2-1) Fluorescent microscopic observation

When the 9 colonies cloned were observed under a fluorescence microscope, GFP-positive cells were observed in all the clones with a positive ratio of 50 to 100%.

### (2-2) FISH analysis

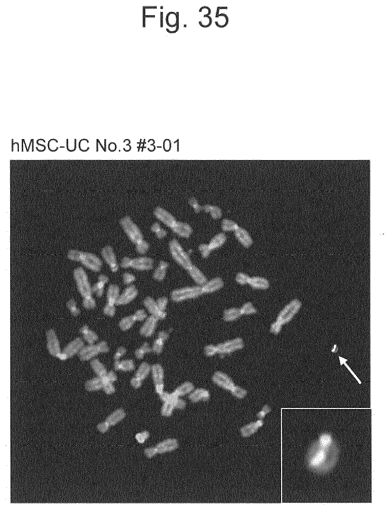

Four clones (clone name: hMSC-UC No. 3 #1-01, #1-02, #2-01, and #3-01) confirmed by PCR analysis were subjected to FISH analysis using PAC harboring HGF (hgf), GDNF (gdnf), IGF-1 (igf-1), and luciferase (e-luc) as a probe. As a result, two clones were confirmed to retain human artificial chromosome without causing karyotypic abnormality (Figure 35, hMSC-UC No. 3, #3-01).

### (3) In vitro induction of differentiation

Osteocyte, chondrocyte, or adipocyte differentiation can be induced as to the four clones confirmed by PCR analysis in accordance with the approach of Okamoto et al. (BBRC, 295: 354, 2002) to confirm whether or not to maintain differentiation potential equivalent to the parent line.

From the analysis results of (1) to (3) described above, it was able to be confirmed that two clones of immortalized MSCs harboring a multi-growth factor-inserted human artificial chromosome vector were obtained by microcell-mediated chromosome transfer.

### (Example 14) Verification of bowel inflammation healing effect of immortalized MSC in inflammatory bowel disease model

### (1) Isolation of CD4-positive, highly CD45RB-positive, and CD25-negative (CD4⁺CD45RB^{High+}CD25⁻) T cell

Thirty 8-week-old female BALB/cAJcl mice (CLEA Japan, Inc.) were acclimatized for 1 week. Then, blood was removed from each mouse by blood collection from the heart under anesthesia, and the spleen and mesenteric lymph nodes were collected. The spleen was subjected to tissue dispersion using MACS system (Miltenyi Biotec) followed by hemolysis treatment. The mesenteric lymph nodes were ground using a plunger for a 1 mL syringe and filtered through a 40 µm cell strainer. The splenocytes and the mesenteric lymph node cells were combined, and CD4-positive cells were separated by treatment with CD4 microbeads (Miltenyi Biotec) and then labeled with antibodies (APC-H7 Rat anti-mouse CD4 antibody (BD), FITC Rat Anti-Mouse CD45RB antibody (BD), and PE/Cy7 anti-mouse CD25 antibody (BioLegend, Inc.)) in order to isolate CD4⁺CD45RB^{High+}CD25⁻ T cells, followed by fractionation with a flow cytometer (MoFlo XDP, Beckman Coulter, Inc.).

### (2) CD4⁺CD45RB^{High+}CD25⁻ T cell transfer

The CD4⁺CD45RB^{High+}CD25⁻T cells were transplanted at 4.0 × 10⁵ cells per animal to 9-week-old female SCID (C.B-17/lcr-scid/scidJcl: CLEA Japan, Inc.) by intravenous injection to the tail (cell transplantation group: 5 groups each involving 8 animals, untreated group (PBS administration): 1 group involving 7 animals). After transplantation, body weight measurement and the observation of coat and feces states were carried out 3 times a week. Random allocation based on change in body weight was performed 21 days after cell administration using the "multivariate block allocation" system of statistical analysis software JMP to group the mice into the cell transplantation groups. In this respect, individuals that fell outside relative body weight mean ± 2SD were excluded.

### (3) Cell culture for transplantation

In order to obtain cells for transplantations, hMSC-UC (parent MSCs) and the immortalized MSCs were cultured under respective cell culture conditions.

### (4) MSC administration (therapeutic cell transplantation)

The parent MSCs or the immortalized MSCs were administered at 1.0 × 10⁶ cells per mouse 21, 28, and 35 days after transplantation of the CD4⁺CD45RB^{High+}CD25- T cells by intravenous injection to the tail. Dexamethasone (Sigma): Dex (1 mg/kg, 100 µL/animal) was subcutaneously administered as a positive control for 14 days from day 21. The Dex solution was prepared before use on the basis of the average body weight of the group on days 21 and 28.

### (5) Sampling

The whole blood was collected 42 days after the start of the experiment, and serum was prepared (preserved at -80°C). The gastrointestinal tract from the pyloric ring to the anus (the colon to the rectum) was further sampled, and state evaluation and weight and length measurement were carried out, followed by fixation in 10% formalin.

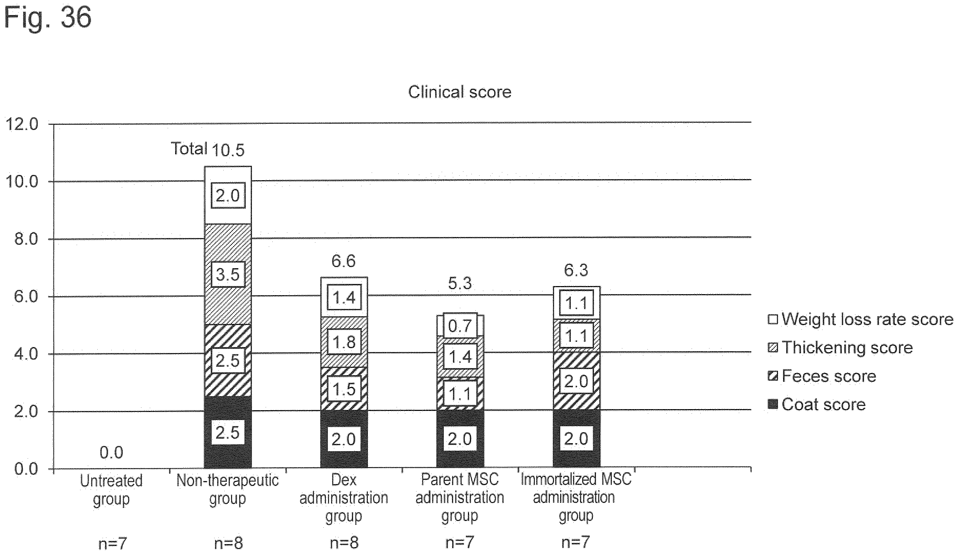

The results are shown in Figure 36. As a result of examining a bowel inflammation therapeutic effect brought about by MSC transplantation in the IBD models of adoptive immunity and transplantation using SCID mice, clinical score values (weight loss score, thickening score, feces score, and coat score values) were drastically decreased in the Dex administration group, the parent MSC administration group, and the immortalized MSC administration group compared with the untreated group. Particularly, for the parent MSC administration group, the score values were decreased to nearly 1/2, and a high effect of improving bowel inflammation was expected. For the immortalized MSC administration group, no marked difference was found from the parent MSC administration group, and the results were equivalent to the Dex administration group. Therefore, the bowel inflammation healing effect of the parent MSC line was found to be maintained even after immortalization and long-term culture through the immortalization. In the culture of the cells for transplantations, the immortalized cells proliferated much better than the cultured early parent cells and had no problem preparing the cells.

### Industrial Applicability

The present invention is applicable to the fields of cell medicine and regenerative medicine. The cell immortalization technique of the present invention is applicable not only to normal cells which proliferate slowly but to cancer cells and the like, and is also applicable to the field of basic research. Furthermore, single-cell cloning essential for gene introduction or chromosome transfer is also achieved. In addition, the cell obtainable by the present invention continues to proliferate without becoming senescent and therefore facilitates quality control, promotes the mechanization of culture, drastically reduces cell production cost, and enables a large number of cells to be handled. Accordingly, the present invention expands the range of application of cell medicine or regenerative medicine to diseases and contributes to the activation of medicine-related industries at home and abroad.

All publications, patents and patent applications cited herein are incorporated herein by reference in their entirety.

## Claims

1. A method for producing a reversibly immortalized cell, comprising the steps of
(1) introducing a chromosomally non-integrated RNA virus vector loaded with an immortalizing gene into a mammalian cell so that the immortalizing gene is expressed in the cell; and
(2) culturing the cell obtained in the step (1) for proliferation.

2. The method according to claim 1, wherein the immortalizing gene is one or two or more immortalizing gene(s) selected from the group consisting of Bmi-1 gene, TERT gene, and SV40T gene.

3. The method according to claim 1 or 2, wherein the immortalizing gene is any of the following (a) to (d):
(a) a combination of Bmi-1 gene, TERT gene, and SV40T gene;
(b) a combination of Bmi-1 gene and TERT gene;
(c) a combination of TERT gene and SV40T gene; and
(d) TERT gene.

4. The method according to any one of claims 1 to 3, wherein the cell is a somatic cell.

5. The method according to claim 4, wherein the somatic cell is a somatic stem cell.

6. The method according to claim 5, wherein the somatic stem cell is a mesenchymal stem cell.

7. The method according to any one of claims 1 to 6, wherein the chromosomally non-integrated RNA virus vector is a negative-strand RNA virus vector.

8. The method according to claim 7, wherein the negative-strand RNA virus vector is a paramyxovirus vector.

9. The method according to claim 8, wherein the paramyxovirus vector is a Sendai virus vector.

10. The method according to claim 9, wherein the Sendai virus vector is a temperature-sensitive Sendai virus vector.

11. The method according to claim 1, wherein the chromosomally non-integrated RNA virus vector is a Sendai virus vector, and the method further comprises the step of removing the Sendai virus vector after culture in the step (2).

12. The method according to claim 11, wherein the removal of the Sendai virus vector is performed by changing a culture temperature from 35°C to 37°C.

13. The method according to any one of claims 1 to 12, further comprising the step of cloning the immortalized cell after culture in the step (2).

14. An immortalized cell obtainable by a method according to any one of claims 1 to 13.

15. An immortalized cell comprising a removable state of a Sendai virus vector loaded with one or two or more immortalizing gene(s) selected from the group consisting of Bmi-1 gene, TERT gene, and SV40T gene.

16. A regenerative medicine product comprising an immortalized cell according to claim 14 or 15.

17. A temperature-sensitive Sendai virus vector loaded with one or two or more immortalizing gene(s) selected from the group consisting of Bmi-1 gene, TERT gene, and SV40T gene.

18. A kit for reversibly immortalized cell preparation comprising a temperature-sensitive Sendai virus vector according to claim 17.
